# FASCICULE DE BREVET EUROPEEN

(11) **EP 0 526 342 B1**
(45) Date de publication et mention de la délivrance du brevet: **04.12.1996**
(21) Numéro de dépôt: 92402200.7
(22) Date de dépôt: 31.07.1992
(51) Int. Cl.: C07D 401/12, C07D 405/14, A61K 31/47

(54) **Isoquinolein-5-yl sulfonamides, leur procédé de préparation et les compositions pharmaceutiques qui les contiennent**
Isochinolin-5-yl-Sulfonsäureamide, Verfahren zu ihrer Herstellung und pharmazeutische Zusammensetzungen, die sie enthalten
Isoquinolin-5yl-sulphonamides, process for their preparation and pharmaceutical compositions containing them

(30) Priorité: 31.07.1991 FR 9109719
(43) Date de publication de la demande: 03.02.1993
(73) Titulaire: ADIR ET COMPAGNIE, F-92415 Courbevoie Cédex (FR)
(72) Inventeur: Peglion, Jean-Louis, F-78110 Le Vesinet (FR); Vilaine, Jean-Paul, F-92290 Chatenay Malabry (FR); Villeneuve, Nicole, F-92500 Rueil Malmaison (FR); Janiak, Philip, F-92110 Clichy (FR)
(74) Mandataire: Reverbori, Marcelle

(56) Documents cités:
- EP-A- 061 673
- EP-A- 0 187 371
- EP-A- 0 287 696
- US-A- 4 857 301
- CHEMICAL ABSTRACTS, vol. 107, no. 25, 21 Décembre 1987, Columbus, Ohio, US; abstract no. 236530b, page 772 ;

## Description

La présente invention a pour objet de nouvelles (isoquinolein-5 yl) sulfonamides, leur procédé de préparation et les compositions pharmaceutiques qui les renferment.

Elle concerne plus particulièrement les composés de formule générale (I): 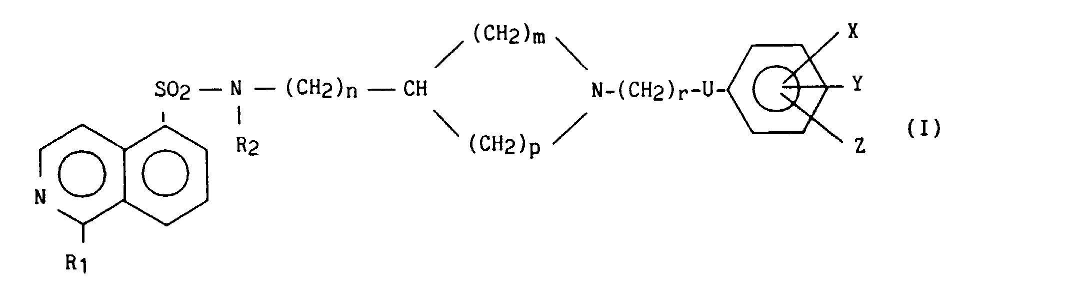 dans laquelle :
n est 0 ou un nombre entier de 1 à 4,
m et p sont des nombres entiers de 1 à 4, étant entendu que la somme m+p est 2,3,4 ou 5,
r est un nombre entier de 1 à 6,

R₁ représente un atome d'hydrogène, un atome de chlore ou un groupement hydroxy,
R₂ représente un atome d'hydrogène, un groupement alkyle inférieur, alkényl inférieur, alkynyle inférieur, cycloalkyle, cycloalkylalkyle inférieur, phényle ou phénylalkyle inférieur,
X, Y, Z, identiques ou différents, représentent un atome d'hydrogène, un atome d'halogène, ou un groupement choisi parmi alkyle inférieur, alcoxy inférieur, nitro, amino, cyano, acétamido, carboxamido, ou X et Y, ou Y et Z forment ensemble, avec les 2 atomes de carbone du noyau phényle qui les portent, un cycle furanique, dihydrofuranique, ou benzénique,
U représente une liaison simple, un atome d'oxygène, un atome de soufre ou un groupement choisi parmi :
   carbonyl, sulfinyl, sulfonyl, -NH-CO-, -CO-NH-, -O-(CH₂)_{r'}-O- avec r' signifiant un nombre entier égal à 2 ou 3, -(CH2)_{r"}-O-(CH₂)_{r"'}-
   avec r" et r"' signifiant des nombres entiers égaux à 1 ou 2, et 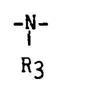 où
   R₃ représente :
      - un atome d'hydrogène,
      - un groupement formyle,
      - un groupement -A, -CO-A, ou -CO-O-A, avec A signifiant un groupement alkyle inférieur, alkényle inférieur, alkynyle inférieur, cycloalkyle, ou cycloalkylalkyle inférieur,
      - un groupement -(CH₂)_{q}-phényl ou un groupement -(CH₂)_{q}-phényl substitué avec q signifiant 0 ou un nombre entier de 1 à 4,
      - un groupement -CO-phényl ou un groupement -CO-phényl substitué,
      - un groupement -CO-O-phényl ou un groupement -CO-O-phényl substitué,
      - un groupement -CO-NR₄R₅.
   où R₄, R₅ :
      - identiques ou différents, représentent un atome d'hydrogène ou un groupement choisi parmi alkyle inférieur, alkényl inférieur, alkynyl inférieur, phényle, phénylalkyle inférieur,
      - soit forment avec l'atome d'azote qui les porte un cycle saturé de 4 à 7 chaînons,
   étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
   étant entendu
      - que les termes "alkyle inférieur" et "alcoxy inférieur" signifient des groupements carbonés saturés linéaires ou ramifiés contenant de 1 à 6 atomes de carbone,
      - que les termes "alkényle inférieur" et "alkynyle inférieur" désignent des groupements insaturés linéaires ou ramifiés contenant de 2 à 6 atomes de carbone,
      - que le terme "cycloalkyl" désigne un cycle carboné saturé contenant de 3 à 8 chaînons.

On connaît de la littérature des composés de structure isoquinoléine sulfonamide montrant notamment une activité antiaggrégante (JP 63-325910), vasodilatatrice (EP 109023), ou bronchorelaxante (USP 4857301).

L'état de l'art antérieur est également constitué des documents suivants : JP 02073067, EP 187371, EP 061673 et BP 0287696.

Des modifications importantes de structure ont conduit aux composés de la présente invention, lesquels présentent une activité pharmacologique et thérapeutique particulièrement puissante et étendue, qu'on ne retrouve pas parmi les composés de l'art antérieur.

En effet, les composés de la présente invention associent à une activité anti-vasoconstrictrice très intéressante un effet protecteur myocardique et périphérique remarquable.

Ces activités sont confirmées par de nombreuses études in vitro aussi bien qu'in vivo (voir exemples de l'étude pharmacologique 31 à 36).

La présente invention a également pour objet le procédé de préparation des composés de formule générale (I) caractérisé en ce que :
- on condense une amine de formule (II) : 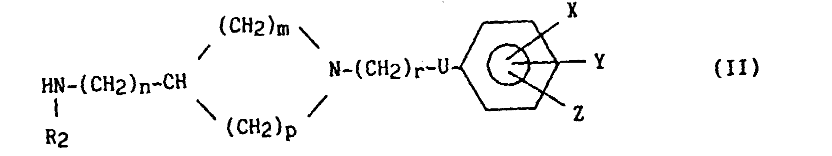 dans laquelle R₂, X, Y, Z, U, n, m, p, et r ont les mêmes significations que dans la formule générale (I),
   avec un composé de formule (III): 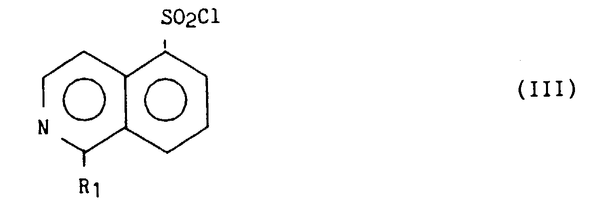 dans laquelle R₁ a la même signification que dans la formule générale (I), pour obtenir un composé de formule (I) : 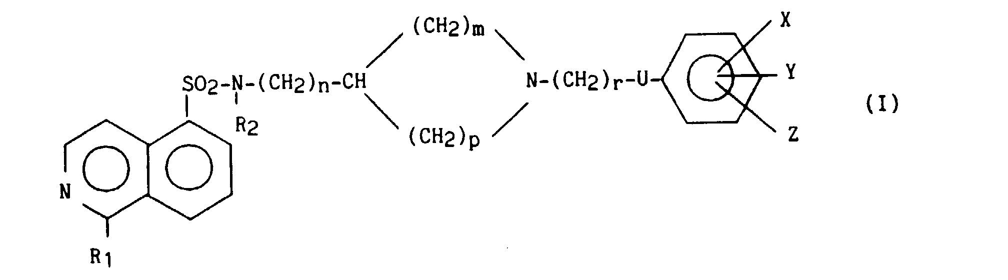 avec R₁, R₂, X, Y, Z, U, n, m, p et r tels que définis précédemment, dont on sépare, le cas échéant, les isomères, et que l'on salifie, si on le désire, avec un acide pharmaceutiquement acceptable,
   composés de formule (I) que l'on purifie, si on le désire, par une technique de cristallisation et/ou de chromatographie.

La présente invention concerne également le procédé de préparation des composés de formule générale (I) caractérisé en ce que :
- on condense une amine de formule (II/a) : 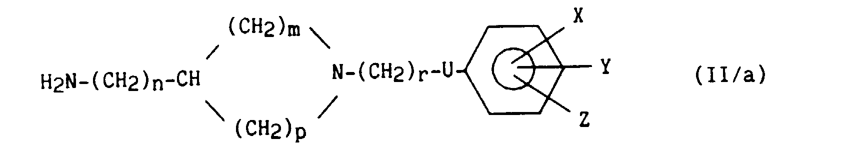 dans laquelle X, Y, Z, U, n, m, p, et r ont les mêmes significations que dans la formule générale (I),
   avec un composé de formule (III) : 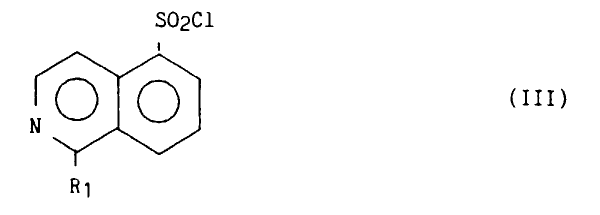 dans laquelle R₁ a la même signification que dans la formule générale (I),
   pour obtenir un composé de formule (I/a) : 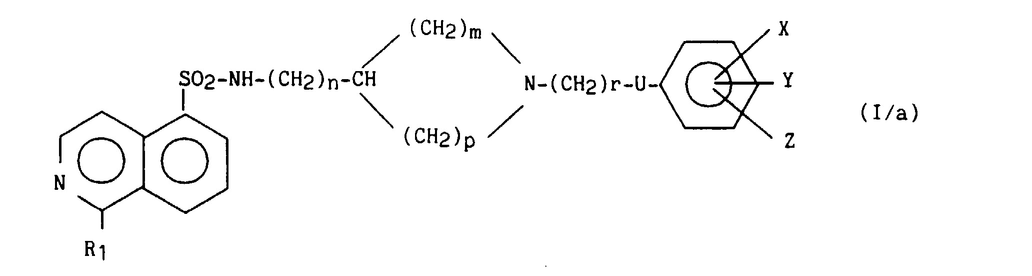 avec R₁, X, Y, Z, U, n, m, p et r tels que définis précédemment, cas particulier des composés de formule (I) où R₂ représente un atome d'hydrogène,
   composé de formule (I/a) que l'on fait réagir avec un composé de formule R′₂-Hal avec R′₂ représentant un groupement alkyle inférieur, alkényle inférieur, alkynyle inférieur, cycloalkyle, cycloalkylalkyle inférieur, phényl ou phénylalkyle inférieur,
   afin d'obtenir un composé de formule (I/b) : 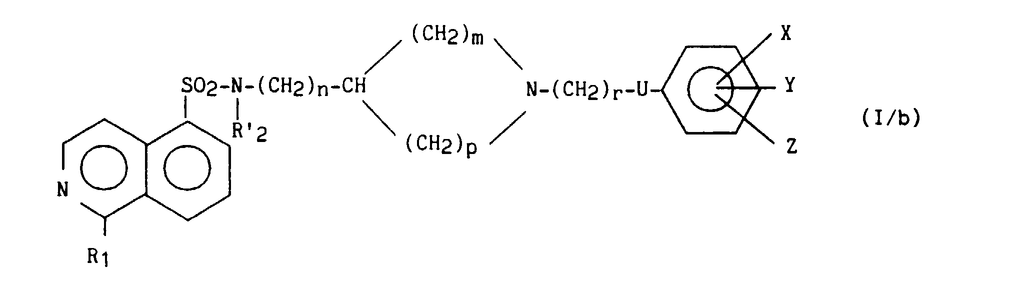 dans laquelle R₁, X, Y, Z, U, n, m, p, r et R′₂ sont tels que définis précédemment, cas particulier des composés de formule (I) où R₂ représente un groupement R′₂.

L'ensemble des dérivés de formule (I/a) et (I/b) forment l'ensemble des dérivés de formule générale (I).

Les composés de formule (I/a) et (I/b) peuvent être séparés, le cas échéant, selon leurs différents isomères, et salifiés, si on le désire, avec un acide pharmaceutiquement acceptable.

Les composés de formule (I/a) et (I/b) peuvent également être purifiés, si on le désire, par une technique de cristallisation et/ou de chromatographie.

La présente invention concerne également un procédé de préparation des composés de formule (I/c) : 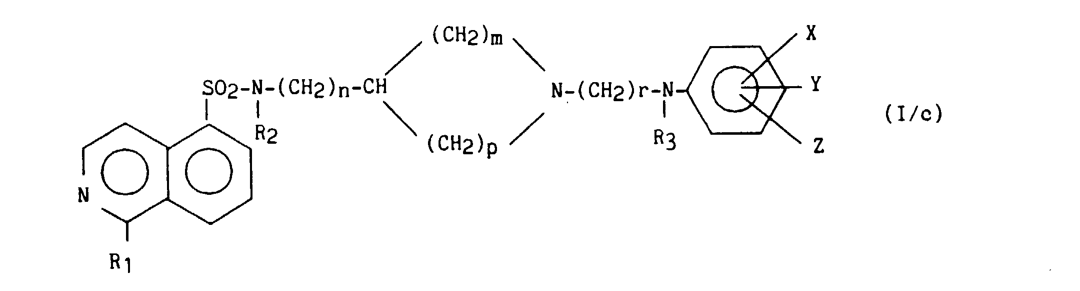 dans laquelle R₁, R₂, R₃, X, Y, Z, n, m, p et r ont la même signification que dans la formule (I),
caractérisé en ce que
- on condense une amine de formule (II/b) : 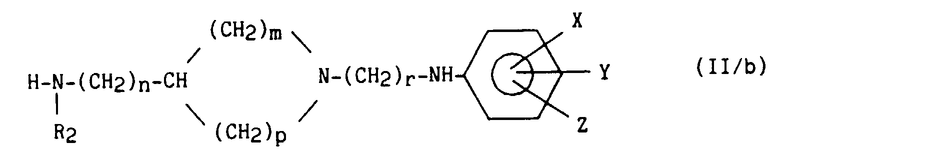 dans laquelle R₂, X, Y, Z, n, m, p et r sont tels que définis précedemment,
- avec un composé de formule (III), 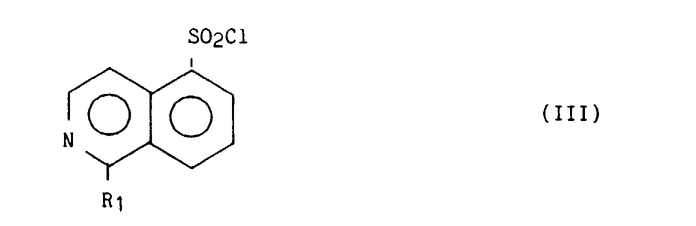 dans laquelle R₁ est tel que défini précédemment,
   pour obtenir un composé de formule (I/d) : 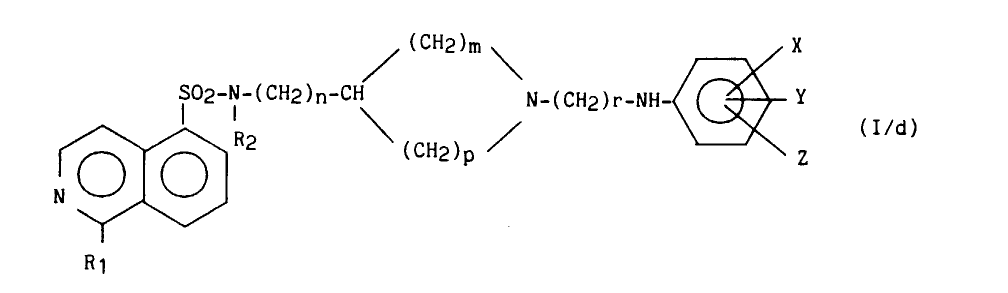 dans laquelle R₁, R₂, X, Y, Z, n, m, p et r sont tels que définis précédemment,
   cas particulier des composés de formule (I/c) où R₃ représente un atome d'hydrogène,
   composé de formule (I/d) que l'on substitue sur l'amine par un groupement de formule R′_{3,} où R′₃ a les mêmes significations que R₃ à l'exception de l'atome d'hydrogène, afin d'obtenir un composé de formule (I/e) : 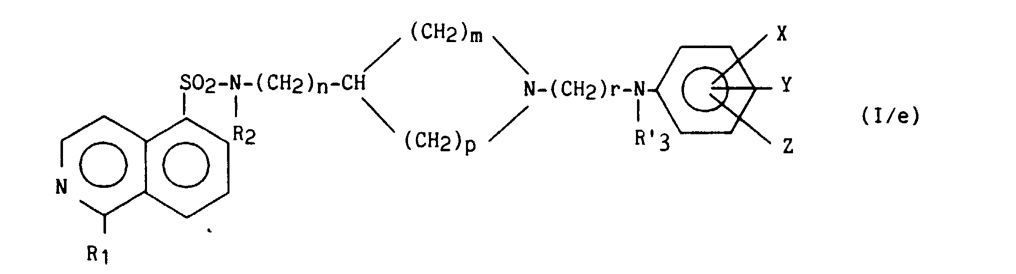 dans laquelle R₁, R₂, X, Y, Z, n, m, p, r et R′₃ sont tels que définis précédemment,
   les composés de formule (I/d) et (I/e) formant l'ensemble des composés de formule (I/c).

Les composés de formule (I/c) peuvent être séparés, le cas échéant, selon leurs différents isomères, et salifiés, si on le désire, avec un acide pharmaceutiquement acceptable.

Les composés de formule (I/c) peuvent également être purifiés, si on le désire, par une technique de cristallisation et/ou de chromatographie.

Les matières premières utilisées dans les procédés précédemment décrits sont soit des produits connus, soit des produits préparés à partir de substances connues, selon des procédés décrits pour préparer des produits analogues comme indiqués dans les exemples suivants.

Les composés de formule générale (I) peuvent être transformés en sels d'addition avec les acides, sels qui font à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthanesulfonique, iséthionique, benzenesulfonique,...

D'autre part, dans le cas où il existe un ou plusieurs carbones asymétriques, les composés de formule (I) peuvent se présenter sous forme de diastéréoisomères ou d'énantiomères, qui font à ce titre, sous forme pure ou sous forme de mélange, partie de l'invention.

Les composés de formule générale (I) et leurs sels d'addition pharmaceutiquement acceptables possèdent des propriétés thérapeutiques intéressantes, notamment dans le domaine cardio-vasculaire.

Les essais pharmacologiques réalisés in vitro ont montré que les composés de l'invention ont en plus d'une activité anti-vasoconstrictrice vis à vis de différents types de médiateurs mettant notamment en jeu la régulation du calcium intracellulaire, une activité protectrice myocardique vis à vis d'une surcharge calcique, de séquences d'ischémie reperfusion ou d'hypoxie réoxygénation.

Les études réalisées in vivo confirment l'activité anti-vasoconstrictrice et l'activité antiischémique de ces composés et mettent en évidence un effet protecteur important vis à vis de lésions vasculaires de nature proliférative.

Ces propriétés permettent donc l'utilisation des composés de la présente invention comme médicament, et notamment dans le domaine cardio-vasculaire dans le traitement et la prévention de l'ischémie myocardique et de ses différentes expressions cliniques comme l'angine de poitrine et l'infarctus du myocarde, mais également dans le traitement des troubles du rythme cardiaque, du spasme vasculaire, de l'hypertension artérielle, des maladies vasculaires et de l'insuffisance cardiaque, et plus généralement dans le traitement et la prévention des troubles liés au vieillissement artériel et à l'athérosclérose.

Ces composés peuvent aussi être administrés pour la prévention des resténoses ou thromboses vasculaires après pontage, dilatation vasculaire notamment coronaire, ou autres formes de reperméabilisation vasculaire. Ils peuvent être également utilisés dans les pathologies métaboliques constituant un facteur de risque cardio-vasculaire tels que l'obésité, le diabète et les dyslipidémies.

En outre, l'effet régulateur du calcium intracellulaire de ces composés leur confère une possible utilisation thérapeutique à titre d'agents antiaggrégants plaquettaires et antithrombotiques ou d'agents relaxants des différents types de muscles lisses (autres que vasculaires déjà cités) : bronchiques, digestifs, urinaires, ou utérins.

De plus de nombreuses situations de souffrance tissulaire qu'elles soient liées au vieillissement, à l'ischémie, à l'inflammation ou à une prolifération cellulaire y compris de nature cancéreuse, peuvent être traitées ou prévenues par les produits de la présente invention.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale, intramusculaire, ou parentérale.

La posologie peut varier notablement selon l'âge et le poids du patient, la voie d'administration, la nature de la maladie et les traitements associés et consiste en prises de 1 à 100 mg, une à plusieurs fois par jour.

Les exemples suivants illustrent l'invention mais ne la limitent en aucune façon.

Les points de fusion sont déterminés à la platine chauffante de Köfler (K) éventuellement sous microscope (MK). Les spectres de résonnance magnétique nucléaire 1H (RMN) ont été réalisés en utilisant le tétraméthylsilane (TMS) comme référence interne. Les déplacements chimiques sont exprimés en partie par million (ppm). Les spectres infrarouge ont été effectués sous forme de pastille de bromure de potassium renfermant environ 1 % du produit à analyser.

### Exemple 1

### Fumarate de N-méthyl N-{1-[4-(p-fluorophénoxy)butyl]pipérid-4 yl} (isoquinoléin-5 yl) sulfonamide.

### Stade A

### 1-bromo 4-(p-fluorophénoxy)butane

Dissoudre 0,5 mole d'hydroxyde de potassium dans 200 cm³ de méthanol. Ajouter 0,5 mole de p-fluorophénol, 3,4 moles de 1,4-dibromobutane, et 3 mmoles d'iodure de potassium. Porter au reflux, sous agitation, pendant 24 heures.
Après évaporation du solvant, reprendre le résidu à l'éther et effectuer un lavage à l'eau puis à l'hydroxyde de sodium 1N. Après avoir éliminé l'excès de composé dibromé, le composé est distillé grâce à un système Kughelrohr.
On obtient une huile qui est le composé attendu :
Eb_{(1,33 Pa)} = 100 °C
Rendement : 66 %.

### Stade B

### N-méthyl N-[1-(benzyl)pypérid-4 yl] acétamide.

Dans une solution contenant 0,2 mole de N-[1-(benzyl)pipérid-4 yl] N-méthyl amine, 0,2 mole de triéthylamine et 500 cm³ de chlorure de méthylène, on ajoute lentement 0,2 mole de chlorure d'acétyle. Laisser en contact 2 heures, évaporer, reprendre à l'éther puis laver à l'eau. Après évaporation, on obtient une huile qui correspond au composé attendu :
Rendement : 80 %.

### Stade C

### N-méthyl N-pipérid-4yl acétamide

Hydrogéner, sous 49.10⁴ Pa de pression d'hydrogène à 50 °C et en présence de 2 grammes d'hydroxyde de palladium, 0,38 mole du composé obtenu au stade B dans 700 cm³ d'éthanol avec 0,38 mole d'acide acétique.
Après filtration et évaporation, reprendre le résidu par 500 cm³ de chlorure de méthylène, puis basifier, sous agitation et à froid, par 0,38 mole d'hydroxyde de sodium 5N.
Décanter , sécher puis évaporer. On obtient le composé attendu.
Rendement : 83 %
Point de fusion : < 50 °C.

### Stade D

### N-méthyl N-{1-[4-(p-fluorophénoxy)butyl]pipérid-4 yl} acétamide.

Porter au reflux un mélange contenant 0,1 mole du composé obtenu au stade A, 0,1 mole du composé obtenu au stade C, et 0,1 mole de carbonate de potassium dans 250 cm³ d'acétone.
Laisser sous agitation durant 12 heures. Après évaporation, reprendre le résidu avec de l'éther puis laver à l'eau. Epuiser la phase éthérée avec de l'acide chlorhydrique 1N, puis basifier à froid les phases acides. Extraire à l'éther, sécher, puis évaporer. On obtient une huile qui correspond au composé attendu et qui sera utilisée ensuite sans purification intermédiaire.
Rendement : 87 %.

### Stade E

### N-{1-[4-(p-fluorophénoxy)butyl}pipérid-4 yl} N-méthyl amine.

Porter au reflux 0,05 mole du composé obtenu au stade D pendant 12 heures en présence de 570 cm³ de méthanol et de 340 cm³ d'acide chlorhydrique 5N. Evaporer le méthanol, verser le mélange sur de la glace puis basifier avec de l'hydroxyde de sodium. Extraire à l'éther.
Après évaporation, on obtient une huile qui correspond au composé attendu. Rendement : 89 %.

### Stade F

### N-méthyl N-{1-[4-(p-fluorophénoxy)butyl]pipérid-4 yl} (isoquinoléin-5 yl) sulfonamide.

A une solution de 0,1 mole du composé obtenu au stade E et de 0,2 mole de diisopropyléthylamine dans 200 cm³ de chlorure de méthylène, ajouter sous agitation et à température ambiante 0,1 mole de chlorhydrate du sulfochlorure de 5-isoquinoléine finement pulvérisé.
Laisser sous agitation pendant 12 heures puis transvaser en ampoule. Laver par 100 cm³ d'hydroxyde de sodium 1N. Sécher, évaporer. L'huile résiduelle est chromatographiée sur 30 fois son poids de silice (AMIKON 60Å) en utilisant comme éluant une solution de chlorure de méthylène/méthanol (95/5) pour obtenir 12 grammes d'une huile qui correspond au composé attendu.

### Stade G

**Le composé du titre.**
Transformer 12 grammes d'huile obtenue au stade F en sel de fumarate par addition de 2,9 grammes d'acide fumarique en solution dans 155 cm³ d'éthanol. On obtient ainsi 10,3 grammes du composé du titre.
Point de fusion : 144-146 °C.
Solvant de cristallisation : éthanol.

### Caractéristiques spectrales

- Infrarouge :: ν(NH/OH) : 2800-2300 cm⁻¹
ν (C=O) : 1685 cm⁻¹
ν(〉N-SO2) : 1325, 1140 cm⁻¹

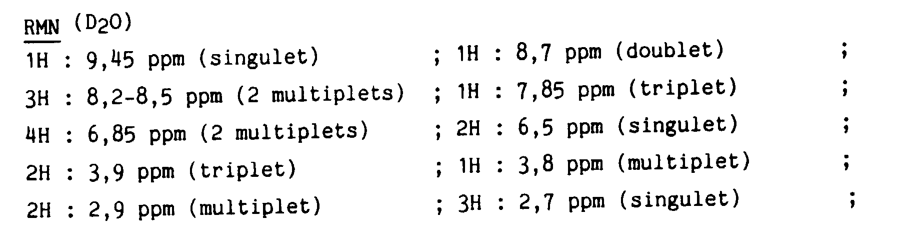

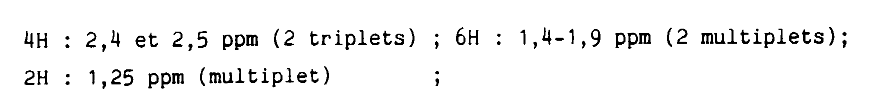

Proton OH et NH⁺ non mis en évidence.

### Exemple 2

### Fumarate de N-méthyl N-{1-[4-(p-nitrophénoxy)butyl]pipérid-4 yl} (isoquinoléin-5 yl) sulfonamide.

En procédant comme dans l'exemple 1 mais en remplaçant au stade A le p-fluorophénol par le p-nitrophénol, on obtient au stade G le composé du titre.
Rendement : 32 %.
Point de fusion : 156-158 °C.
Solvant de cristallisation : méthanol.

### Exemple 3

### Fumarate de N-méthyl N-{1-[4-(phénoxy)butyl]pipéryd-4 yl} (isoquinoléin-5yl) sulfonamide.

En procédant comme dans l'exemple 1 mais en remplaçant au stade A le p-fluorophénol par le phénol, on obtient au stade G le composé du titre.
Point de fusion : 176-178 °C.
Solvant de cristallisation : méthanol.

### Exemple 4

### Fumarate de N-méthyl N-{1-[4-(p-méthylphénoxy)butyl] pipérid-4 yl} (isoquinoléin-5 yl) sulfonamide.

En procédant comme dans l'exemple 1 mais en remplaçant au stade A le p-fluorophénol par le p-crésol, on obtient au stade G le composé du titre.
Rendement : 31 %.
Point de fusion : 160-163 °C.
Solvant de cristallisation : éthanol.

### Exemple 5

### Fumarate de N-méthyl N-{1-[4-(napht-1 yloxy)butyl]pipérid-4 yl} (isoquinoléin-5 yl) sulfonamide.

En procédant comme dans l'exemple 1 mais en remplaçant au stade A le p-fluorophénol par le 1-naphtol, on obtient au stade G le composé du titre.
Rendement : 32 %.
Point de fusion : 184-186 °C.
Solvant de cristallisation : éthanol.

### Exemple 6

### Chlorhydrate de N-méthyl N-{1-[4-(p-chlorophénoxy)butyl]pipérid-4 yl} isoquinoléin-5 yl) sulfonamide.

En procédant comme dans l'exemple 1 mais en remplaçant au stade A le p-fluorophénol par le p-chlorophénol et en remplaçant au stade G de l'exemple 1 l'acide fumarique par l'acide chlorydrique, on obtient le composé du titre.
Point de fusion : 194-196 °C.
Solvant de cristallisation : méthanol.

### Exemples 7 à 11

En suivant le procédé décrit dans l'exemple 6 mais en remplaçant au stade A de l'exemple 1 le p-fluorophénol par les composés adéquats, on obtient successivement les composés des titres suivants :

### Exemple 7

### Chlorhydrate de N-méthyl N-{1-[4-(p-méthoxyphénoxy)butyl]pipérid-4 yl} (isoquinoléin-5 yl) sulfonamide.

Point de fusion : 176-179 °C.
Solvant de cristallisation : acétonitrile.

### Exemple 8

### Dichlorhydrate de N-méthyl N-{1-[4-(p-cyanophénoxy)butyl]pipérid-4 yl} (isoquinoléin-5 yl) sulfonamide.

Point de fusion : 224-226 °C.
Solvant de cristallisation : acétonitrile.

### Exemple 9

### Dichlorhydrate de N-méthyl N-{1-[4-(2,3-dihydrobenzofuran-5 yloxy)butyl]pipérid-4 yl} (isoquinoléin-5 yl) sulfonamide.

Rendement : 40 %.
Point de fusion : 194-197 °C.
Solvant de cristallisation : éthanol.

### Exemple 10

### Dichlorhydrate de N-méthyl N-{1-[4-(m-fluorophénoxy)butyl] pipérid-4 yl} (isoquinoléin-5 yl) sulfonamide.

Point de fusion : 185-186 °C.
Solvant de cristallisation : acétonitrile

### Exemple 11

### Dichlorhydrate de N-méthyl N-{1-[4-(o-fluorophénoxy)butyl]pipérid-4 yl} (isoquinoléin-5 yl) sulfonamide.

Rendement : 39,5 %.
Point de fusion : 200-203 °C.
Solvant de cristallisation : acétonitrile.

### Exemple 12

### Dichlorhydrate de N-méthyl N-{1-[4-(p-fluorophénylthio)butyl]pipérid-4 yl} (isoquinoléin-5 yl) sulfonamide.

En procédant comme dans l'exemple 1, mais en remplaçant au stade A le p-fluorophénol par le 4-fluorothiophénol et en remplaçant au stade F l'acide fumarique par l'acide chlorhydrique, on obtient le composé du titre.
Point de fusion : 145 °C.
Solvant de cristallisation : acétate d'éthyle.

### Exemple 13

### Dichlorhydrate de N-{1-[4-(p-fluorophénoxy)butyl]pipérid-4 yl} (isoquinoléin-5 yl) sulfonamide.

En procédant comme dans l'exemple 1, mais en remplaçant au stade B la N-[1-(benzyl)pipérid-4 yl] N-méthyl amine par la 1-benzyl 4-aminopipéridine, et en remplaçant au stade G l'acide fumarique par l'acide chlorhydrique, on obtient le composé du titre.
Point de fusion : 160 °C.
Solvant de cristallisation : acétonitrile.

### Exemple 14

### N-{1-[5-(p-fluorophénoxy)pentyl]pipérid-4 yl} (isoquinoléin-5 yl) sulfonamide.

En procédant comme dans l'exemple 1, mais en remplaçant au stade A le 1,4-dibromobutane par le 1,5-dibromopentane, au stade B la N-[1-(benzyl) pipérid-4 yl] N-méthyl amine par la 1-benzyl 4-aminopipéridine, on obtient successivement :

### Stade A

### 1-bromo 5-(p-fluorophénoxy)pentane.

Rendement : 49 %.
Eb_{(10,66 Pa)} = 120 °C**.**

### Stade B

### N-[1-(benzyl)pipérid-4 yl]acétamide.

### Stade C

### N-(pipérid-4 yl)acétamide.

### Stade D

### N-{1-[5-(p-fluorophénoxy)pentyl]pipérid-4 yl}acétamide.

Rendement : 48 %.
Point de fusion : 133-134 °C**.**

### Stade E

### 4-amino 1-[5-(p-fluorophénoxy)pentyl]pipéridine.

Rendement : 65 %.

### Stade F

### Le composé du titre.

Point de fusion : 142-145 °C.
Solvant de cristallisation : acétonitrile**.**

### Caractéristiques spectrales :

### Infrarouge :

ν (NH) : 3309 cm⁻¹
ν (NH-SO₂) : 1321,1215 cm⁻¹

### RMN (CDCl₃) :

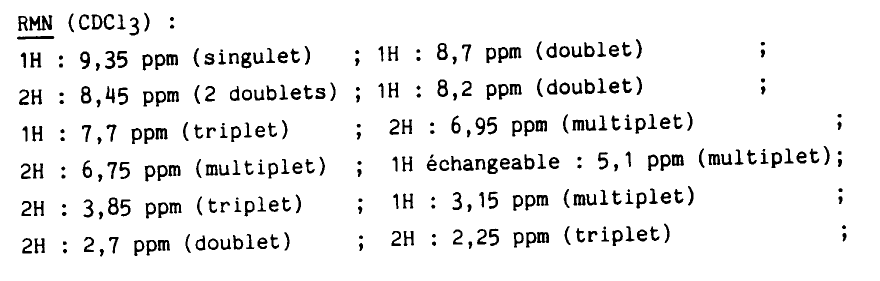

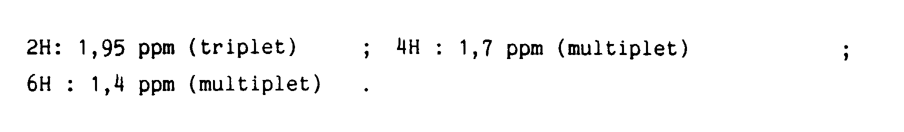

### Exemple 15

### N-{[1-[3-(p-fluorophénoxy)propyl]pipérid-4 yl]méthyl} (isoquinoléin-5 yl) sulfonamide.

### Stade A

### 1-bromo 3-(p-fluorophénoxy)propane.

En procédant comme au stade A de l'exemple 1 mais en remplaçant le 1,4-dibromobutane par le 1,3-dibromopropane, on obtient le composé attendu.
Rendement : 43 %.
Eb_{(1,33 Pa)} = 85 °C.

### Stade B

### {1-[3-(P-fluorophénoxy)propyl]pipérid-4 yl}carboxamide.

Dissoudre 46,2 grammes du composé obtenu au stade A et 25,6 grammes d'isonipécotamide dans 560 cm³ d'acétone en présence de 27,6 grammes de carbonate de potassium. Porter à reflux sous agitation pendant 12 heures. Evaporer, reprendre à l'éther puis laver à l'eau. Extraire la phase éthérée avec de l'acide chlorhydrique 1N puis basifier les phases aqueuses à froid. Filtrer le précipité et le sécher. On obtient le composé désiré.
Point de fusion : 140-142 °C.

### Stade C

### 4-aminométhyl 1-[3-(p-fluorophénoxy)propyl]pipéridine.

Ajouter, par l'intermédiaire d'une ampoule à solide, 3,8 grammes d'hydrure de lithium et d'aluminium à une suspension de 26 grammes du composé obtenu au stade B. Porter à reflux pendant 2 heures. Décomposer le milieu réactionnel par 2,6 cm³ de H₂O, puis 2,1 cm³ d'hydroxyde de sodium et enfin 9,6 cm³ de H₂O. Filtrer puis évaporer le filtrat.
On obtient 13,1 grammes d'une huile qui correspond au composé attendu et que l'on peut utiliser sans autre purification.

### Stade D

### Le composé du titre.

A un mélange de 13 grammes du composé obtenu au stade C et 17,8 cm³ de diisopropyléthylamine dans 500 cm³ de chlorure de méthylène, on ajoute, par fractions et sous agitation à température ambiante, 13 grammes de chlorhydrate du sulfochlorure de 5-isoquinoléine. Maintenir 12 heures sous agitation. Transvaser dans une ampoule à décanter puis laver par 50cm³ d'hydroxyde de sodium 1N. Sécher et évaporer. Le résidu est chromatographié sur silice avec pour éluant un mélange chlorure de méthylène/méthanol (95/5). Concrétiser l'huile obtenue par l'acétonitrile.
On obtient 4,5 grammes du composé du titre.
Point de fusion : 123-125 °C.
Solvant de cristallisation : acétonitrile.

### Exemple 16

### N-méthyl N-{2-[1-[2-(p-fluorophénoxy)éthyl]pipérid-4 yl] éthyl} (isoquinoléin-5 yl) sulfonamide.

En suivant un processus analogue à celui de l'exemple 15, mais en remplaçant au stade C la 4-aminométhyl 1-[3-(p-fluorophénoxy)propyl]pipéridine par la 4-[2-(amino)éthyl] 1-[2-(p-fluorophénoxy)éthyl]pipéridine, on prépare le composé du titre.

### Exemple 17

### Dichlorhydrate de N-méthyl N-{[1-[3-(p-fluorophénoxy)propyl]pipérid-4 yl] méthyl} (isoquinoléin-5 yl) sulfonamide.

### Stade A

### N-méthyl N- {[1-[3-(p-fluorophénoxy)propyl] pipérid-4 yl] méthyl} (isoquinoléin-5 yl) sulfonamide.

Couler une suspension de 6 grammes du composé de l'exemple 15 dans 30 cm³ de diméthylacétamide sur une suspension de 0,6 g d'hydrure de sodium à 60% dans 20 cm³ de diméthylacétamide.
Ajouter 1,8 g d'iodure de méthyle et laisser 12 heures à température ambiante. Diluer à l'eau, extraire à l'acétate d'éthyle et sécher. Purifier par chromatographie sur silice en utilisant comme éluant un mélange chlorure de méthylène/méthanol (95/5).
On obtient 5,5 g d'une huile qui correspond au composé attendu.

### Stade B

### Le composé du titre.

Reprendre les 5,5 g du composé obtenu au stade A dans 10 cm³ d'acétonitrile. Ajouter à cette solution 7,5 cm³ d'une solution 3,3 N d'éther chlorhydrique.
On obtient 3,1 g d'un solide qui correspond au composé du titre.
Point de fusion : 146-149 °C.
Solvant de cristallisation : acétonitrile.

### Caractéristiques spectrales :

### Infrarouge :

v (NH⁺) : 2515 cm⁻¹

### RMN (DMSO d6)

| | |
|---|---|
| 1H : 9,55 ppm (singulet) ; | 1H : 8,75 ppm (doublet) ; |
| 1H : 8,5 ppm (doublet) ; | 2H : 8,45 ppm (multiplet) ; |
| 1H : 7,9 ppm (triplet) ; | 2H : 7,15 ppm (triplet) ; |
| 2H : 6,95 ppm (multiplet) ; | 2H : 4,05 ppm (triplet + multiplet) ; |
| 11H : 3,6-2,8 ppm ; | 7H :2,2-1,3 ppm . |

### Exemple 18

### N-{[1-[3-(phénoxy)propyl]pipérid-4 yl]méthyl} (isoquinoléin-5 yl) sulfonamide.

En procédant comme dans l'exemple 15 mais en remplaçant dans le stade A le p-fluorophénol par le phénol, on obtient au stade D le composé du titre.
Rendement : 39 %.

### Exemple 19

### N-méthyl N-{[1-[3-(phénoxy)propyl]pipérid-4 yl]méthyl} (isoquinoléin-5 yl) sulfonamide.

En procédant comme dans le stade A de l'exemple 17, mais en remplaçant le composé de l'exemple 15 par le composé de l'exemple 18, on obtient le produit du titre.
Point de fusion : 99-101 °C.
Solvant de cristallisation : cyclohexane.

### Exemple 20

### Dichlorhydrate de N-méthyl N-{[1-[4-(p-fluorophényl)butyl]pipérid-4 yl]méthyl} (isoquinoléin-5 yl) sulfonamide.

### Stade A

### {1-[4-(p-fluorophényl)butyl]pipérid-4 yl}carboxylate d'éthyle.

En procédant comme au stade B de l'exemple 15 mais en remplaçant l'isonipécotamide par l'isonipécotate d'éthyle et le composé du stade A par le 4-fluorophényl 1-iodobutane, on obtient le composé attendu.

### Stade B

### {1-[4-(p-fluorophényl)butyl]pipérid-4 yl} N-méthylcarboxamide

Le composé obtenu au stade précédent est saponifié avant d'être dissout dans le chlorure de méthylène. On le traite par du carbonyldiimidazole et après 1 heure de contact (fin du dégagement gazeux), on coule une solution de méthylamine dans le chlorure de méthylène en respectant la stoechiométrie. On laisse en contact une nuit, lave à l'eau, sèche, évapore le solvant.

### Stade C

### 1-[4-(p-fluorophényl)butyl] 4-[(méthylamino)méthyl]pipéridine.

Le composé du stade précédent est réduit par action de l'hydrure de lithium et d'aluminium selon un mode opératoire identique à celui du stade C de l'exemple 15.

### Stade D

### Le composé du titre

En utilisant un mode opératoire identique à celui des stades F et G de l'exemple 1 mais en remplaçant dans le stade F, le composé du stade E de l'exemple 1 par le composé du stade précédent et dans le stade G de l'exemple 1 l'acide fumarique par l'acide chlorhydrique, on obtient le composé désiré.
Rendement : 32 %.
Point de fusion : 182 °C.
Solvant de cristallisation : acétate d'éthyle.

### Exemple 21

### Dichlorhydrate de N-méthyl N-{[1-[4-(p-fluorophénoxy)butyl]pyrrolidin-3 yl]méthyl} (isoquinoléin-5 yl) sulfonamide.

### Stade A

### N-méthyl (1-benzyl 2-oxopyrrolidin-4 yl)carboxamide.

Dissoudre 0,2 mole d'acide (1-benzyl 2-oxopyrrolidin-4 yl) carboxylique (décrit dans Journal of Organic Chemistry, 1961 ; 26 : p 1519) dans 200 cm³ de chlorure de méthylène. Traiter par 32,4 g de carbonyldiimidazole. Laisser en contact 1 heure, puis ajouter une solution composée de 20 g de méthylamine et de 200 cm³ de chlorure de méthylène. Laisser en contact pendant 12 heures à température ambiante, laver à l'eau puis sécher sur sulfate de magnésium.
On obtient une huile qui est le composé attendu.
Rendement : 92 %.

### Stade B

### N-{[1-(benzyl)pyrrolidin-3 yl]méthyl} N-méthyl amine

Réduire 40 g du composé obtenu au stade A à l'aide de 13,1 g d'hydrure de lithium et d'aluminium.
On obtient 33,6 g du composé du stade B sous forme huileuse.
Rendement : 89 %.

### Stade C

### N-méthyl N-{[1-(benzyl)pyrrolidin-3 yl]méthyl}acétamide.

Dissoudre 33,6 g du composé obtenu au stade B dans 350 cm³ de chlorure de méthylène et 21,6 cm³ de triéthylamine. Acétyler par 10,9 cm³ de chlorure d'acétyle. Laisser 2 h en contact, évaporer, reprendre dans l'éther, laver à l'eau puis sécher. Chromatographier sur silice l'huile obtenue après évaporation en utilisant comme éluant le mélange chlorure de méthylène/méthanol (95/5).
On obtient 12,5 g du composé désiré.

### Stade D

### Acétate de N-méthyl N-[(pyrrolidin-3 yl)méthyl]acétamide.

Hydrogéner 0,05 mole du composé obtenu au stade C dans 130 cm³ d'éthanol et 3 cm³ d'acide acétique en présence de 1,3 g de dihydroxyde de palladium à 50 °C et sous 5 kilogrammes/cm² de pression. Evaporer puis filtrer.
On obtient 12 g du composé attendu.

### Stade E

### N-méthyl N-{[1-[4-(p-fluorophénoxy)butyl]pyrrolidin-3 yl]méthyl}acétamide.

En procédant comme dans le stade D de l'exemple 1 mais en remplaçant le composé du stade C par le composé obtenu au stade D de l'exemple 21, on obtient le composé désiré.
Rendement : 38 %.

### Stade F

### N-{[1-[4-(p-fluorophénoxy)butyl]pyrrolidin-3 yl]méthyl} N-méthyl amine.

Porter à reflux 8,5 g du composé obtenu au stade E pendant 20 heures en présence de 80 cm³ d'acide chlorhydrique concentré, de 80 cm³ d'eau et de 260 cm³ de méthanol. Evaporer, verser sur de la glace, basifier à l'hydroxyde de sodium puis extraire à l'éther. Après évaporation, on obtient le composé souhaité sous forme d'huile.
Rendement : 78 %.

### Stade G

### Le composé du titre.

En procédant de façon anologue aux stades F et G de l'exemple 1, mais en utilisant le composé obtenu au stade précédent, et en utilisant l'éther chlorhydrique au lieu de l'acide fumarique, on obtient le produit du titre.
Rendement : 28 %.

### Exemple 22

### Dichlorhydrate de N-méthyl N-{[1-[3-(p-fluorophénoxy)propyl]pyrrolidin-3yl]méthyl} (isoquinoléin-5 yl) sulfonamide.

En procédant comme dans l'exemple 21, mais en remplaçant lors du stade E le 1-bromo 4-(p-fluorophénoxy)butane par le 1-bromo 3-(p-fluorophénoxy)propane, on obtient le composé du titre.
Rendement : 51 %.
Point de fusion : 145 °C.
Solvant de cristallisation : acétate d'éthyle

### Caractéristiques spectrales :

### Infrarouge :

ν (OH) : 3400 cm⁻¹
ν (NH⁺) : 2800 - 1800 cm⁻¹
ν (NH-SO2) : 1377, 1142 cm⁻¹

### Exemple 23

### Dichlorhydrate de N-méthyl N-{1-[5-(p-fluorophénoxy) 3-oxapentyl]pipérid-4 yl} (isoquinoléin-5 yl) sulfonamide.

### Stade A

### 1-chloro 5-(p-fluorophénoxy) 3-oxapentane.

En procédant comme au stade A de l'exemple 1 mais en remplaçant le 1,4-dibromobutane par le 1,5-dichloro 3-oxapentane, on obtient le produit désiré.
Rendement : 53 %.

### Stade B

### 5-(p-fluorophénoxy) 1-iodo 3-oxapentane.

Porter à reflux pendant 24 heures un mélange de 0,1 mole du composé obtenu au stade A et de 0,12 mole d'iodure de sodium dans 100 cm³ de méthyléthylcétone. Evaporer le solvant, reprendre à l'éther, laver à l'eau puis au thiosulfate de sodium 1N. sécher puis évaporer. On obtient sous forme huileuse le composé attendu.
Rendement : 90 %.

### Stade C

### Le composé du titre.

En procédant de façon analogue aux stades D à G de l'exemple 1, mais en utilisant à la place du composé du stade A de l'exemple 1, le composé du stade B précédent et en remplaçant au stade G de l'exemple 1 l'acide fumarique par l'éther chlorhydrique, on obtient le composé du titre.
Rendement : 47 %.
Point de fusion : 210-212 °C.
Solvant de cristallisation : acétonitrile.

### Exemple 24

### Fumarate de N-{[1-[3-(p-fluorophénylamino)propyl]pipérid-4 yl]méthyl} (isoquinoléin-5 yl) sulfonamide.

### Stade A

### 1-bromo 2-(fluorophénylaminocarbonyl)éthane.

Préparer une solution de 0,1 mole de p-fluoroaniline, 14 cm³ de triéthylamine et de 100 cm³ de benzène. Ajouter goutte à goutte, et à température ambiante un mélange de 17,1 g du chlorure de l'acide 3-bromo propionique et de 20 cm³ de benzène. Laisser 12 h sous agitation puis transvaser dans une ampoule à décanter. Sécher, puis évaporer pour obtenir 21 g du composé attendu.
Point de fusion : 136-138 °C.

### Stade B

### {1-[2-(p-fluorophénylaminocarbonyl)éthyl]pipérid-4 yl}carboxamide.

En procédant comme au stade B de l'exemple 15, mais en remplaçant le composé obtenu au stade A de l'exemple 15 par le composé obtenu au stade A précédent, on obtient le composé désiré.
Rendement : 38 %.

### Stade C

### 4-aminométhyl 1-[3-(p-fluorophénylamino)propyl] pipéridine.

Mettre en suspension 9,5 g du composé obtenu au stade précédent dans 300 cm³ de tétrahydrofurane. Introduire, par l'intermédiaire d'une ampoule à solide, 0,064 mole d'hydrure de lithium et d'aluminium. Ajouter de nouveau 100 cm³ de tétrahydrofurane et porter à reflux pendant 12 heures. Après traitement du milieu réactionnel, on obtient 5,5 g d'une huile correspondant au composé désiré.
Rendement : 65 %.

### Stade D

### Le composé du titre.

En procédant comme au stade D de l'exemple 15 et après salification par l'acide fumarique dans l'éthanol, on obtient le composé du titre.
Point de fusion : 168-170 °C.
Solvant de cristallisation : acétonitrile.

### Exemple 25

### Fumarate de N-méthyl N-{1-[3-(p-fluorophénylamino)propyl]pipérid-4 yl] méthyl} (isoquinoléin-5 yl) sulfonamide.

### Stade A

### 4-éthoxycarbonyl 1-{[3-(p-fluorophénylamino) 3-oxo]propyl}pipéridine.

Dissoudre 24,6 g du composé obtenu au stade A de l'exemple 24 et 15,7 g d'isonipécotate d'éthyle dans 360 cm³ d'acétone en présence de 13,8 g de carbonate de potassium. Puis procéder de façon analogue à celle du stade B de l'exemple 16 pour obtenir le produit désiré.
Rendement : 56 %.

### Stade B

### {1-[3(p-fluorophénylamino) 3-oxopropyl]pipérid-4 yl} N-méthyl carboxamide.

Mettre en suspension 92 g du composé obtenu au stade A préalablement saponifié dans 500 cm³ de chlorure de méthylène. Ajouter en une seule fois 0,313 mole de carbonyldiimidazole. Laisser en contact pendant 2 h, au cours desquelles on note un passage progressif en solution. Puis ajouter goutte à goutte une solution de 33 g de méthylamine dans 400 cm³ de chlorure de méthylène.
Après avoir laissé en contact 48 h, transvaser dans une ampoule à décanter puis laver à l'eau. Concentrer de moitié, puis filtrer le précipité qui correspond au composé attendu.
Rendement : 46 %.

### Stade C

### Le composé du titre.

En procédant comme aux stades C et D de l'exemple 24 mais en utilisant le composé obtenu au stade B précédent, on obtient le composé du titre.
Point de fusion : 174-176 °C.
Solvant de cristallisation : méthanol.

### Exemple 26

### Dichlorhydrate de N-éthyl N-{1-[4-(p-fluorophénoxy)butyl]pipérid-4 yl} (isoquinoléin-5 yl) sulfonamide.

En procédant comme dans le stade A de l'exemple 17 mais en remplaçant le composé de l'exemple 15 par le composé de l'exemple 13 et l'iodure de méthyle par l'iodure d'éthyle, on obtient après salification par l'acide fumarique dans l'éthanol le composé du titre.
Rendement : 32,5 %.
Point de fusion : 154-156 °C.
Solvant de cristallisation : acétonitrile.

### Exemple 27

### Dichlorhydrate de N-méthyl N-{[1-[3-(N-acétyl p-fluorophénylamino) propyl]pipérid-4 yl]méthyl} (isoquinoléin-5 yl) sulfonamide.

Dissoudre 0,01 mole du composé obtenu à l'exemple 25 et 0,011 mole d'anhydride acétique dans 10 cm³ d'acide acétique. Porter à reflux sous agitation pendant 3 heures. Verser dans 150 cm³ d'eau puis basifier par de la soude à 20 %. Extraire au chlorure de méthylène. Sécher puis évaporer. Dissoudre l'huile obtenue dans 10 cm³ d'acétate d'éthyle et salifier par de l'éther chlorhydrique. Précipiter puis filtrer.
On obtient le composé du titre.
Rendement : 27 %.
Point de fusion : 135 °C.
Solvant de cristallisation : acétate d'éthyle.

### Exemple 28

### Chlorhydrate de N-méthyl N-{[1-[3-(N-formyl p-fluorophénylamino) propyl]pipérid-4 yl]méthyl} (isoquinoléin-5 yl) sulfonamide.

Dissoudre 4,6 g du composé obtenu à l'exemple 25 préalablement chauffé à 40 °C dans 24,6 cm³ d'acide formique à 88 %. Ajouter 8,8 cm³ d'anhydride acétique. Laisser en contact pendant 48 h. Verser sur de la glace, basifier, puis extraire à l'acétate d'éthyle. Après chromatographie sur silice en utilisant comme éluant le mélange chlorure de méthylène/méthanol (95/5), évaporer et reprendre par 20 cm³ d'acétate d'éthyle. Ajouter ensuite 2,2 cm³ d'éther chlorhydrique 3N. Refroidir puis filtrer le précipité qui correspond au composé attendu.
Point de fusion : 176-178 °C.
Solvant de cristallisation : acétate d'éthyle.

### Exemples 29 à 34

En procédant par analogie avec les méthodes précédemment décrites on été préparés les produits objets des exemples 29 à 34 ci-après :

### Exemple 29

### Dichlorhydrate de N-méthyl N-{[1-[3-(N-éthoxycarbonyl)p-fluorophénylamino)propyl]pipérid-4 yl]méthyl} (isoquinoléin-5 yl) sulfonamide.

### Exemple 30

### Chlorhydrate de N-méthyl N-{[1-[3-(N-aminocarbonyl) p-fluorophénylamino) propyl]pipérid-4 yl]méthyl} (isoquinoléin-5 yl) sulfonamide.

### Exemple 31

### Dichlorhydrate de N-{1-[3-(N-p-fluorophényl N-méthylamino)propyl]piperid-4 yl méthyl} (isoquinoléin-5 yl)sulfonamide.

PF (K) : 158 °C.

### Exemple 32 :

### N-méthyl N-{1-[-3-(p-fluorophénylcarbonyl)propyl]pipérid-4 yl méthyl} (isoquinoléin-5 yl)sulfonamide.

PF (MK) : 118-120 °C.

### Exemple 33 :

### dichlorhydrate de N-méthyl N-{1-[3-(N-p-fluorophényl N-méthylamino) propyl]piperid-4 yl méthyl} (isoquinoléin-5 yl)sulfonamide.

PF (K) : 225-227 °C.

### Exemple 34 :

### Dichlorhydrate deN-{2-[1-[2-(p-fluorophénoxy)éthyl]pipérid-4-yl]éthyl} (isoquinoléin-5 yl) sulfonamide.

PF (K) : 158-160 °C.

### Exemple 35

### Etude pharmacologique des composés de l'invention

Les propriétés pharmacologiques et thérapeutiques très intéressantes des composés de l'invention sont revelées par de nombreux tests pharmacologiques.

Ces test seront présentés dans l'ordre suivant :
I Etudes in vitro des composés de l'invention
   A/ activité antivasoconstrictrice
   B/ effet protecteur myocardique
II Etudes in vivo des composés de l'invention
   A/ effet sur la réactivité cardio-vasculaire
   B/ étude de l'action hémodynamique
   C/ effet sur les arrythmies de reperfusion
   D/ effet sur l'index de prolifération après dénudation endothéliale.

### I ETUDES IN VITRO DES COMPOSÉS DE L'INVENTION

### A/ activité anti-vasoconstrictrice

### Matériel et méthodes

Les études sont réalisées sur des anneaux de 3 mm de longueur
- d'aortes prélevées chez des rats WISTAR (325-375 g) anesthésiés au pentobarbital sodique (30 mg/kg en intrapéritonéal)
- d'aortes, d'artères mésentériques, et d'artères iliaques prélevées chez des lapins de Nouvelle-Zélande (1,8-2 kg) également anesthésiés au pentobarbital sodique (30 mg/kg en intrapéritonéal)
- ou d'artères coronaires de porcs anesthésiés au nesdonal (8 mg/kg en intraveineux).

Les anneaux vasculaires sont immergés dans une solution physiologique, normale ou dépourvue de calcium (+ 0,2mM du chélateur EGTA), thermostatée à 37 °C, aérée par un mélange 95 % O₂ + 5 % CO₂. Les anneaux sont reliés à un capteur de tension STATHAM (UC2-GOULD). Les tensions optimales pour chaque vaisseau sont appliquées et une période de stabilisation de 90 mn des préparations est respectée.

### a) Activité anti-vasoconstrictrice des composés de l'invention en milieu physiologique.

### Protocoles d'étude

- L'activité relaxante des composés de l'invention, testés en concentrations cumulatives additionnées toutes les 15 minutes, vis à vis de la contraction d'anneaux vasculaires induite par un milieu hyperpotassique (80 mM KCl, 37 mM NaCl), la noradrénaline (10⁻⁶ M), l'endothéline (6.10⁻⁹ M) ou un ester de phorbol (TPA/phorbol 12 myristate-13 acétate à 10⁻⁵ M), a permis le calcul de l'IC₅₀ (Concentration molaire qui inhibe de 50 % la contraction maximale).
- L'activité des composés de l'invention, préincubés pendant 15 minutes, est également appréciée par la limitation de la contraction vasculaire induite par le PDGF (Platelet Derived Growth Factor) à 3.10⁻¹¹ M, par l'acétylcholine (3.10⁻⁸ à 3.10⁻⁵ M) ou par des séquences de stimulation électrique.

### Résultats

Le tableau (T1) regroupe les IC50 obtenus avec les composés exemplifiés représentatifs des composés de l'invention vis à vis de la contraction d'aorte de rat induite par un milieu hyperpotassique.

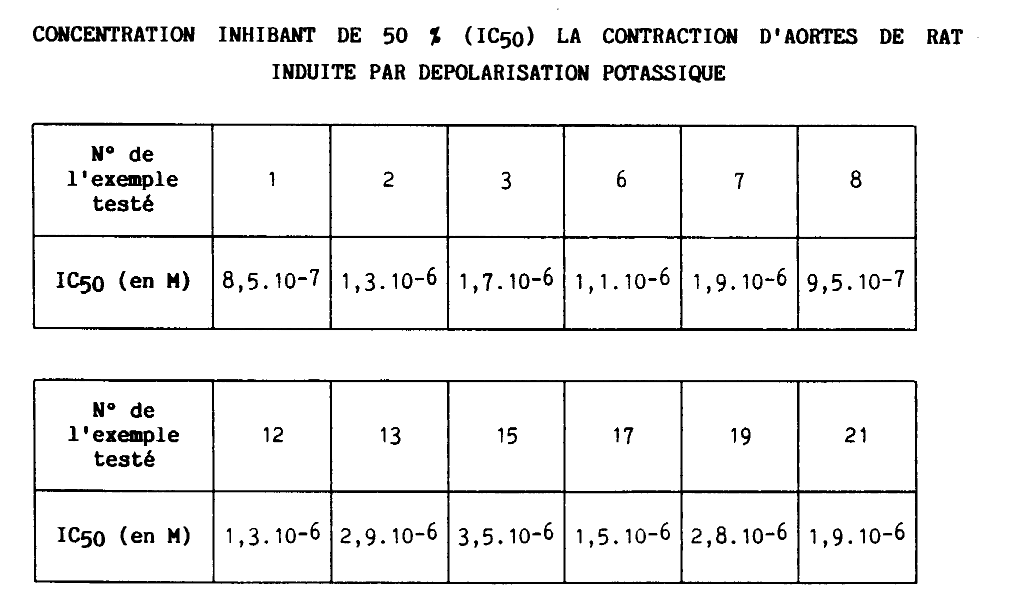

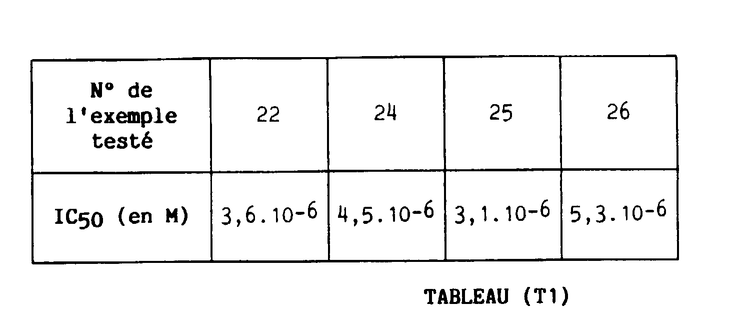

Les tableaux (T2) et (T3) montrent l'activité des composés des exemples 1 et 17 représentatifs des composés de l'invention vis à vis des autres stimuli vaso-constricteurs testés.

### ACTIVITE ANTI-VASOCONSTRICTRICE DES COMPOSES DE L'INVENTION EN MILIEU PHYSIOLOGIQUE NORMAL

**(T2)**

| **STIMULUS** | **VAISSEAU** | **COMPOSE DE L'EXEMPLE 1 IC**_{**50**} **(M)** | **COMPOSE DE L'EXEMPLE 17 IC**_{**50**} **(M)** |
|---|---|---|---|
| **Noradrénaline** | aorte de lapin | 1,4.10⁻⁶ | 1,5.10⁻⁶ |
| **Stimulation électrique** | artère mésentérique de lapin | 4,0.10⁻⁷ | 1,0.10⁻⁷ |
| **Endothéline** | aorte de lapin | 8,1.10⁻⁶ | 3,3.10⁻⁶ |
| **Phorbol ester** | aorte de lapin | 3,0.10⁻⁴ | 2,1.10⁻⁵ |

**(T3)**

| **STIMULUS** | **VAISSEAU** | **% D'INHIBITION DE LA CONTRACTION à 10**^{**-5**} **M** | |
|---|---|---|---|
| | | **COMPOSE DE L'EXEMPLE 1** | **COMPOSE DE L'EXEMPLE 17** |
| **PDGF** | Aorte de lapin | 20 | 30 |
| **Acétylcholine** | artère coronaire de porc | 61 | 64 |

### b) Activité anti-vasoconstrictrice des composés de l'invention en milieu sans calcium.

### Protocoles d'étude

L'effet inhibiteur d'une concentration des composés de l'invention incubée pendant 30 minutes, vis à vis d'une contraction vasculaire induite par la noradrénaline (10⁻⁶ M), l'angiotensine (10⁻⁵ M), l'endothéline (3.10⁻⁸ M) ou la caféine (10 mM) en milieu dépourvu de calcium, est testé.

### Résultats

Le tableau (T4) montre l'activité des composés vis à vis de la contraction d'aortes de lapin induite par la noradrénaline en milieu dépourvu de calcium.

### % D'INHIBITION DE LA CONTRACTION INDUITE PAR LA NORADRENALINE EN MILIEU SANS CALCIUM

**(T4)**

| **N° de l'exemple testé** | **Concentration du composé** | |
|---|---|---|
| | **10**^{**-6**} **M** | **10**^{**-7**} **M** |
| **1** | **61** | |
| **2** | **12** | |
| **3** | **47** | |
| **6** | **18** | |
| **7** | **13** | |
| **12** | **19** | |
| **13** | **42** | |
| **15** | **59** | |
| **17** | **90** | **40** |
| **19** | **67** | **31** |
| **21** | **73** | **17** |
| **22** | **82** | **43** |
| **24** | **67** | **38** |
| **25** | **77** | **22** |
| **26** | **26** | |

Le tableau (T5) montre l'activité des composés des exemples 1 et 17 représentatifs des composés de la présente invention vis à vis des autres stimuli vasoconstricteurs testés également en milieu dépourvu de calcium.

**(T5)**

| **STIMULUS** | **VAISSEAU** | **% D'INHIBITION DE LA CONTRACTION à 10**^{**-5**} **M** | |
|---|---|---|---|
| | | **COMPOSE DE L'EXEMPLE 1** | **COMPOSE DE L'EXEMPLE 17** |
| **Angiotensine II** | Aorte de lapin | 40 | 21 |
| **Endothéline** | Aorte de lapin | 42 | 67 |
| **Ester de phorbol** | Aorte de lapin | 66 | 35 |
| **Caféine** | Artère iliaque de lapin | 30 | 63 |

### CONCLUSION

Les dérivés de l'invention exercent une activité inhibitrice remarquable vis à vis de la contraction vasculaire induite par différents types de médiateurs vasoconstricteurs (tels : noradrénaline, angiotensine II, acétylcholine, endothéline) ou par un facteur de croissance (tel le PDGF) qui mettent en jeu notamment dans la cellule musculaire lisse une entrée de calcium et une mobilisation de calcium intra-cellulaire. De façon très intéressante ces produits conservent une activité en absence de calcium extra-cellulaire. Ils sont de plus actifs sur les tests mettant directement en jeu une activation de la protéine kinase C ( ester de phorbol) ou une mobilisation du calcium intra-cellulaire (caféine).

### B/ Etude de l'activité protectrice des composés de l'invention sur le myocarde

### a) Effet des composés de l'invention sur une intoxication de l'oreillette gauche de cobaye

### Protocole d'étude

On prélève les oreillettes gauches sur des cobayes (350-450 g) anesthésiés au pentobarbital sodique (30 mg/kg). Les oreillettes sont accrochées à un capteur de tension STATHAM (UC2-GOULD) et la tension initiale appliquée est de 0,5 grammes.
On stimule les oreillettes électriquement à 1 Hz par l'intermédiaire d'électrodes de platine.
Les intoxications sont réalisées soit par une addition de ouabaïne (10⁻⁶ M), soit par une addition de vératridine (10⁻⁵ M).
On ajoute les composés à tester 15 minutes avant l'addition de l'agent toxique.

Les résultats sont présentés dans les tableaux (T6) et (T7).

### EFFET DES COMPOSES DE L'INVENTION SUR UNE INTOXICATION A LA OUABAÏNE DE L'OREILLETTE GAUCHE DE COBAYE

**(T6)**

| | **SOLVANT** | **COMPOSE DE L'EXEMPLE 1 10**^{**-6**} **M** | **COMPOSE DE L'EXEMPLE 17 10**^{**-5**} **M** |
|---|---|---|---|
| **% CONTRACTION** | | | |
| **10 MIN** | 201 ± 17,9 | 192 ± 29 | 201,5 ± 31,5 |
| **30 MIN** | 63,7 ± 15 | 187,5 ± 30 | 186,5 ± 46 |
| **60 MIN** | 45 ± 10 | 148,3 ± 33 | 179 ± 44 |

| **CONTRACTURE (%)** | | | |
|---|---|---|---|
| **15 MIN** | 13,2 ± 7,7 | 0 | 0 |
| **30 MIN** | 57,8 ± 13,7 | 1,9 ± 1,9 | 2,5 ± 2,5 |
| **60 MIN** | 75 ± 12 | 18 ± 7 | 5 ± 3,3 |

### EFFET DES COMPOSES DE L'INVENTION SUR UNE INTOXICATION A LA VERATRIDINE DE L'OREILLETTE GAUCHE DE COBAYE

**(T7)**

| | **SOLVANT** | **COMPOSE DE L'EXEMPLE 1 10**^{**-5**} **M** | **COMPOSE DE L'EXEMPLE 17 10**^{**-5**} **M** |
|---|---|---|---|
| **% CONTRACTION** | | | |
| **5 MIN** | 227,4 ± 30,6 | 158 ± 11 | 218,8 ± 50,8 |
| **15 MIN** | 30,4 ± 3,7 | 60,5 ± 13 | 181 ± 51 |
| **60 MIN** | 28,9 ± 5,3 | 20,5 ± 4,6 | 157,1 ± 42,6 |

| **CONTRACTURE (%)** | | | |
|---|---|---|---|
| **15 MIN** | 42,7 ± 9,7 | 23,5 ± 6,2 | 0 |
| **30 MIN** | 81,7 ± 13,4 | 52,2 ± 4,7 | 0 |
| **60 MIN** | 44,9 ± 8,3 | 60,7 ± 4,4 | 0 |

Ces tests pharmacologiques montrent que les composés de la présente invention s'opposent de façon importante au développement de la contracture et à l'effondrement de la contraction liés aux effets toxiques de la ouabaïne et de la vératridine.

Ainsi, après une intoxication de 30 minutes à la ouabaïne, la contracture des oreillettes de cobaye n'est pas significative en présence des composés de l'invention alors qu'elle dépasse 50 % de la contraction développée en leur abscence. Parallèlement, la contractilité est préservée en présence des composés de l'invention alors qu'elle est réduite de 70 % dans les expériences contrôles.

### b) Effet protecteur des composés de l'invention sur un coeur isolé soumis à une séquence ischémie-reperfusion.

### Protocole d'étude

On prélève le coeur sur des rats WISTAR (325-375 g). Le coeur est rapidement perfusé selon la technique de LANGENDORFF à une pression constante de 101,3 x10² Pa et stimulé électriquement à 5 Hz.
Le coeur est soumis à une ischémie de 30 mn (réalisée par arrêt total de la perfusion) suivie par une reperfusion de 60 mn. Les composés à tester sont mis en incubation 15 mn avant l'ischémie.

Les contractions isovolumétriques sont enregistrées par l'intermédiaire d'un ballonet en polyéthylène relié à un capteur de pression (P23-GOULD) introduit dans le ventricule gauche et gonflé de manière à obtenir une pression diastolique comprise entre 6,7.10² et 13,3.10² Pa.

### Résultats

Les composés de l'invention limitent le développement de la contracture au cours de la période ischémique et permettent une meilleure récupération fonctionnelle au cours de la reperfusion.
A titre d'exemple, le composé de l'exemple 1 permet à une concentration de 8.10⁻⁷ M de réduire de 47 % par rapport aux expériences contrôles la contracture développée au bout de 30 minutes d'ischémie et améliore la récupération fonctionnelle des coeurs de 55 % par rapport aux expériences contrôles au terme des 60 minutes de reperfusion.

### c) Effet protecteur des composés de l'invention sur un coeur isolé soumis à une hypoxie-réoxygénation.

On suit un protocole expérimental analogue à celui décrit dans le test précédent, mais en remplaçant l'étape d'ischémie-reperfusion par une hypoxie de 60 mn réalisée par l'administration d'un mélange gazeux 95 % N₂ + 5 % CO₂ pendant cette période et suivie par une réoxygénation de 30 mn.

Les composés à tester sont mis en incubation 15 mn avant et pendant la durée de l'hypoxie.

### Résultats

Les composés de la présente invention limitent également dans ces conditions le développement de la contracture au cours de l'hypoxie et permettent une meilleure récupération fonctionnelle au cours de la réoxygénation. Ainsi le composé de l'exemple 17 réduit de 42 % la contracture développée lors d'expériences contrôles et augmente de plus de 100 % la contraction par rapport aux coeurs témoins au bout de 30 minutes de réoxygénation.

### CONCLUSION

On montre donc par ces études sur le tissu myocardique que les composés de la présente invention possèdent un effet protecteur puissant comme cela est montré vis à vis d'une surcharge de calcium intracellulaire (intoxication par la ouabaïne ou la vératridine) ou vis à vis d'agressions telles que l'ischémie-reperfusion ou l'hypoxie réoxygénation.

### II ETUDE IN VIVO

Des études ont été réalisées in vivo pour vérifier l'activité anti-vasoconstrictrice et protectrice des composés de la présente invention vis à vis d'agression du myocarde et prouver leur effet protecteur vis à vis de lésions vasculaires de nature proliférative.

### A/ Effet des composés de l'invention sur la réactivité cardiovasculaire

### a) Effets des composés de l'invention administrés par voie intraveineuse sur la réactivité cardiovasculaire à différents agents vasopresseurs.

### Protocole d'étude

Après une période de stabilisation de 30 à 60 mn de rats WISTAR vigiles, une gamme de concentrations de phényléphrine (entre 0 et 16 µg/kg) est injectée par voie veineuse.
La lecture de la pression artérielle moyenne et de la fréquence cardiaque s'effectue à l'instant où l'effet presseur atteint son maximum. Après une période de lavage d'un minimum de 2 heures, le composé à tester est administré par voie intraveineuse (10 mg/kg) suivi 15 minutes après par une nouvelle gamme de phényléphrine.

Le même protocole est utilisé pour l'étude des composés de l'invention sur les effets presseurs induits par l'angiotensine II (0 à 200 ng/kg) et la vasopressine (0 à 200 ng/kg). Ces études se déroulent sur des groupes d'animaux différents.

### Résultats

Les composés de la présente invention réduisent de façon très significative les réponses pressives induites par les différents agents vasopresseurs utilisés.

A titre d'exemple, le composé de l'exemple 17, lorsqu'il est administré en intraveineuse à une dose de 10 mg/kg, réduit de 81 % l'effet presseur de la phényléphrine.

### b) Effets des composés de l'invention administrés par voie orale sur la réactivité cardiovasculaire à la phényléphrine.

### Protocole d'étude

On procède comme dans le protocole précédent, mais on remplace l'administration intraveineuse des composés à tester par une administration orale (5, 10 et 20 mg/kg) du composé à tester. La gamme de phénylephrine est répétée 0,5 1, 2, 3, 4, 5 et 6 heures après l'administration du composé.

### Résultats

Après administration orale des composés de l'invention la réponse pressive est significativement diminuée par rapport au groupe témoin. L'analyse des différents temps d'étude montrent que les composés de l'invention ont une bonne activité par voie orale et une longue durée d'action.

A titre d'exemple, l'activité inhibitrice du composé de l'exemple 17 apparaît 30 mn après son administration, atteint son maximum au bout de 2 heures (60 % de réduction de l'effet vasopresseur de la phényléphrine à une dose de 20 mg/kg), et se maintient pendant 6 heures.

### B/ Etude de l'action hémodynamique des composés de la présente invention chez le rat.

### Protocole d'étude

Après anesthésie au pentobarbital (60 mg/kg, en intrapéritonéal), les animaux sont instrumentés en vue de la mesure de la pression artérielle, de la fréquence cardiaque et des vélocités sanguines rénales, mésentériques et musculaires.
Les rats sont ventilés artificiellement à 50 cycle/mn. Après une période de stabilisation, on administre des doses cumulatives des composés à tester, à 15 mn d'intervalle par voie intraveineuse.

### Résultats

L'activité anti-vasoconstrictrice des composés de l'invention se traduit par une baisse de pression artérielle liée à une vasodilatation périphérique et une réduction de la fréquence cardiaque chez ces animaux dont le tonus sympathique est augmenté par la procédure d'anesthésie.

### C/ Action des composés de l'invention sur les arrythmies de reperfusion.

### Protocole d'étude

On pratique sur des rats WISTAR une thoracotomie et une ligature coronarienne. Après 10 mn de stabilisation, on administre 2 mg/kg en intraveineuse du composé à tester.

La pression artérielle, la fréquence cardiaque et l'électrocardiogramme sont enregistrés. Cinq minutes après l'injection du produit, la ligature coronarienne est serrée.

L'ischémie myocardique induite par cette sténose complète se manifeste par une diminution de la pression artérielle et un élévation du segment ST de l'électrocardiogramme. Après 5 minutes de sténose, la ligature est retirée et l'électrocardiogramme ainsi que la pression artérielle sont enregistrées pendant les 10 minutes consécutives. Le délai d'apparition des différents troubles du rythme, ainsi que la mortalité sont mesurés.

### Résultats

La reperfusion du territoire cardiaque ischémié engendre très rapidement l'apparition de troubles du rythme : extrasystoles ventriculaires, tachycardies ventriculaires, et fibrillations ventriculaires qui peuvent entrainer la mort de l'animal (4 morts sur 9 dans le groupe témoin).
Les composés de l'invention présentent un très bon effet protecteur contre les troubles du rythme de reperfusion.
A titre d'exemple, l'injection intraveineuse du composé de l'exemple 17 retarde très significativement l'apparition des extrasystoles et des tachycardies ventriculaires.
Chez les animaux traités par le composé de l'exemple 17, le taux de mortalité est nul et l'apparition des fibrillations ventriculaires est prévenu à 100 %.

### D/ Effet des composés de l'invention sur l'index de prolifération après dénudation endothéliale

### Protocole d'étude

On prétraite pendant 6 jours des rats WISTAR mâles de 300 g avec 10 mg/kg quotidiens par voie orale du composé à tester.
Après 6 jours de prétraitement (J6), une dénudation de l'endothélium aortique des animaux est réalisée selon la méthode suivante :
Après anesthésie de l'animal au méthoxital (Briétal, 60 mg/kg en intrapéritonéal), une sonde à embolectomie (Fogarty 2F) est introduite dans l'aorte par voie carotidienne gauche.
La déendothélialisation aortique est réalisée par trois passages successifs de la sonde.

Trois jours après la dénudation endothéliale (J9), les rats sont sacrifiés et l'aorte est prélevée, incubée dans une solution physiologique pendant 1 heure à 37 °C, puis transférée dans une solution de Krebs-Henseleit enrichie en ³H thymidine (activité spécifique : 1,48-2,22 TBq/moles) pendant 1 heure à 37 °C. Une post-incubation d'une heure suit dans une solution de Krebs-Henseleit dépourvue de ³H-thymidine.
Après lavage dans un tampon Tris-EDTA, l'index de prolifération est calculé à partir de l'incorporation de ³H-thymidine.

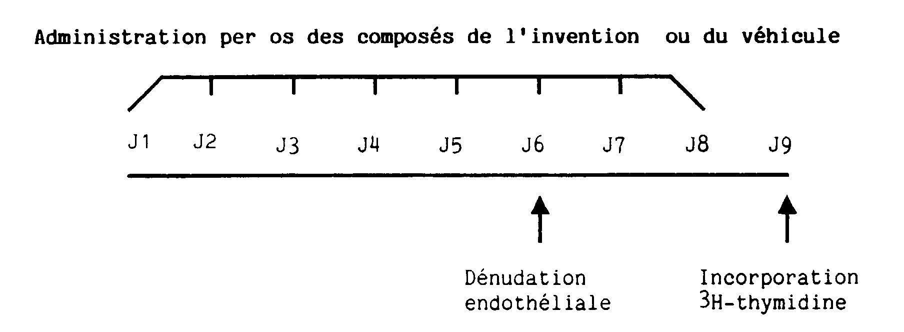

### Résultats

Les composés de la présente invention diminuent significativement l'index de prolifération des cellules musculaires lisses d'aorte de rat ayant subi une dénudation endothéliale.
A titre d'exemple, les composés des exemples 1 et 17 administrés par voie orale réduisent de 40 % la prolifération des cellules musculaires lisses.

### CONCLUSION

Les études réalisées in vivo confirment les activités anti-vasoconstrictrices et antiischémiques remarquables des composés de la présente invention et mettent en évidence un effet protecteur important vis à vis du processus de prolifération des cellules musculaires lisses vasculaires qui constitue une étape fondamentale du développement des lésions vasculaires et notamment des lésions athéromateuses et des resténoses après désobstruction vasculaire.

## Revendications (Revendications pour l'(les) Etat(s) contractant(s) suivant(s): AT, BE, CH, DE, DK, FR, GB, IT, LI, LU, NL, PT, SE)

1. Les composés de formule générale (I) : 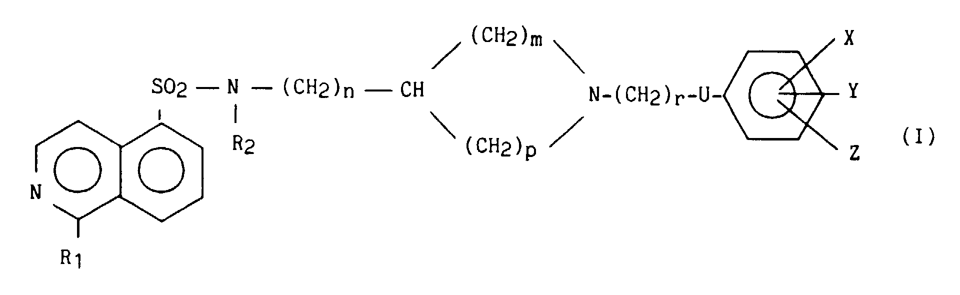 dans laquelle :
n est 0 ou un nombre entier de 1 à 4,
m et p sont des nombres entiers de 1 à 4,
étant entendu que la somme m+p est 2,3,4 ou 5,
r est un nombre entier de 1 à 6,
R₁ représente un atome d'hydrogène, un atome de chlore ou un groupement hydroxy,
R2 représente un atome d'hydrogène, un groupement alkyle inférieur, alkényl inférieur, alkynyle inférieur, cycloalkyle, cycloalkylalkyle inférieur, phényle ou phénylalkyle inférieur,
Y, Y, Z, identiques ou différents, représentent un atome d'hydrogène, un atome d'halogène, ou un groupement choisi parmi alkyle inférieur, alcoxy inférieur, nitro, amino, cyano, acétamido, carboxamido, ou X et Y, ou Y et Z forment ensemble avec les 2 atomes de carbone du noyau phényle qui les portent, un cycle furanique, dihydrofuranique, ou benzénique,
U représente une liaison simple, un atome d'oxygène, un atome de soufre ou un groupement choisi parmi :
carbonyl, sulfinyl, sulfonyl, -NH-CO-, -CO-NH-, -O-(CH₂)_{r′}-O- avec r' signifiant un nombre entier égal à 2 ou 3, -(CH₂)_{r"}-O-(CH₂)_{r"'}-
avec r" et r"' signifiant des nombres entiers égaux à 1 ou 2, et 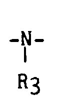 où
R₃ représente :
- un atome d'hydrogène,
- un groupement formyle,
- un groupement -A, -CO-A, ou -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué avec q signifiant 0 ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅
où R₄, R₅ :
- identiques ou différents, représentent un atome d'hydrogène ou un groupement choisi parmi alkyle inférieur, alkényl inférieur, alkynyl inférieur, phényle, phénylalkyle inférieur,
- soit forment avec l'atome d'azote qui les porte un cycle saturé de 4 à 7 chaînons,
étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
étant entendu
- que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkynyle inférieur" désignent des groupements insaturés linéaires ou ramifiés contenant de 2 à 6 atomes de carbone,
- que le terme "cycloalkyl" désigne un cycle carboné saturé contenant de 3 à 8 chaînons.
leurs éventuels isomères optiques, ainsi que le cas échéant, leurs sels d'addition à un acide pharmaceutiquement acceptable.

2. Les composés de formule (I) selon la revendication 1 dans lesquels m et p représentent chacun 2,
ainsi que, le cas échéant, leurs sels d'addition avec un acide pharmaceutiquement acceptable.

3. Le composé selon la revendication 1 qui est le N-méthyl N-{1-[4-(p-fluorophénoxy)butyl]pipérid-4 yl} (isoquinoléin-5 yl)sulfonamide,
ainsi que ses sels d'addition avec un acide pharmaceutiquement acceptable.

4. Le composé selon la revendication 1 qui est le N-méthyl N-{[1-[3-(p-fluorophénoxy)propyl]pipérid-4 yl]méthyl} (isoquinoléin-5 yl) sulfonamide,
ainsi que ses sels d'addition à un acide pharmaceutiquement acceptable.

5. Le composé selon la revendication 1 qui est le N-méthyl N-{[1-[3-(p-fluorophénoxy)propyl]pyrrolidin-3 yl]méthyl} (isoquinoléin-5 yl) sulfonamide,
ses isomères optiques ainsi que ses sels d'addition avec un acide pharmaceutiquement acceptable.

6. Le composé selon la revendication 1 qui est le N-{[1-[3-(p-fluorophénylamino)propyl]pipérid-4 yl]méthyl } (isoquinoléin-5 yl) sulfonamide, ainsi que ses sels d'addition avec un acide pharmaceutiquement acceptable.

7. Le composé selon la revendication 1 qui est le N-méthyl N-{[1-[4-(p-fluorophényl)butyl]pipérid-4 yl]méthyl} (isoquinoléin-5 yl) sulfonamide, ainsi que ses sels d'addition à un acide pharmaceutiquement acceptable.

8. Le procédé de préparation des composés de formule (I) selon la revendication 1, caractérisé en ce que :
- on condense une amine de formule (II) : 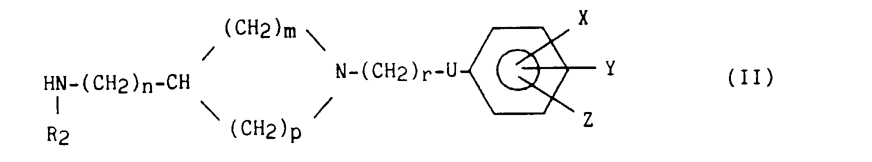 dans laquelle R₂, X, Y, Z, U, n, m, p, et r ont les mêmes significations que dans la formule générale (I),
avec un composé de formule (III) : 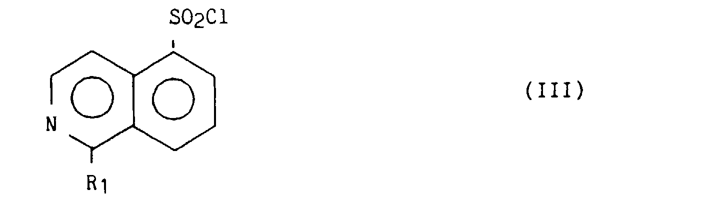 dans laquelle R₁ a la même signification que dans la formule générale (I), pour obtenir un composé de formule (I) : 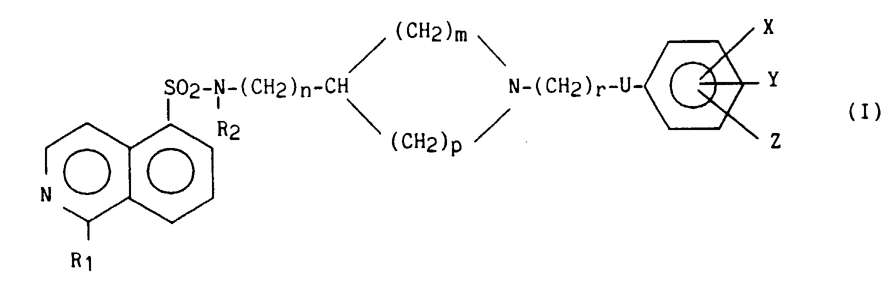 avec R₁, R₂, X, Y, Z, U, n, m, p et r tels que définis précédemment, dont on sépare, le cas échéant, les isomères, et que l'on salifie, si on le désire, avec un acide pharmaceutiquement acceptable, composés de formule (I) que l'on purifie, si on le désire, par une technique de cristallisation et/ou de chromatographie.

9. Le procédé de préparation des composés de formule (I) selon la revendication 1, caractérisé en ce que :
- on condense une amine de formule (II/a) : 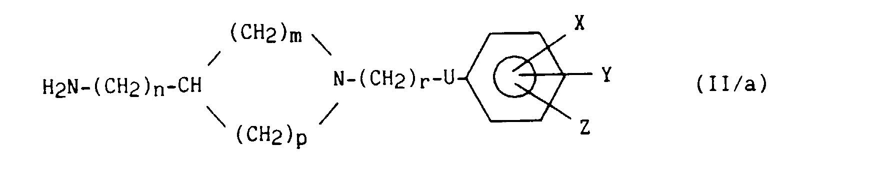 dans laquelle X, Y, Z, U, n, m, p, et r ont les mêmes significations que dans la formule générale (I),
avec un composé de formule (III) : 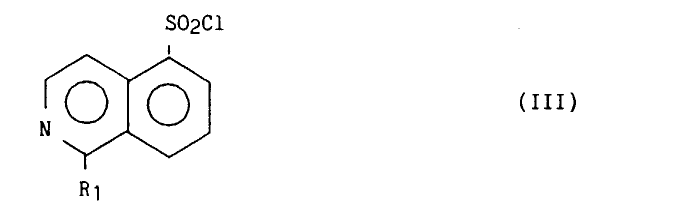 dans laquelle R₁ a la même signification que dans la formule générale (I),
pour obtenir un composé de formule (I/a) : 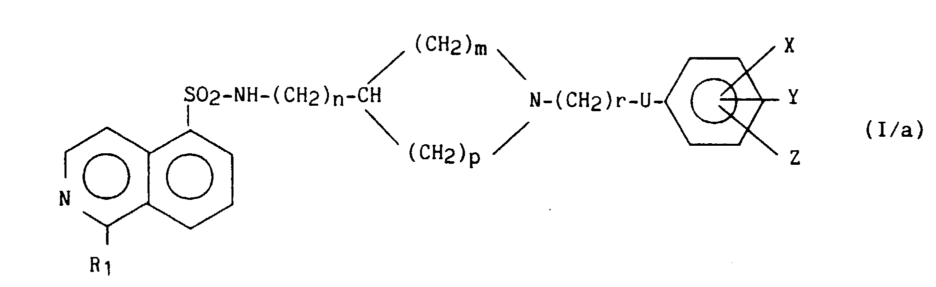 avec R₁, X, Y, Z, U, n, m, p et r tels que définis précédemment, cas particulier des composés de formule (I) où R₂ représente un atome d'hydrogène,
composé de formule (I/a) que l'on fait réagir avec un composé de formule R'₂-Hal avec R'₂ représentant un groupement alkyle inférieur, alkényle inférieur, alkynyle inférieur, cycloalkyle, cycloalkylalkyle inférieur, phényl ou phénylalkyle inférieur,
afin d'obtenir un composé de formule (I/b) : 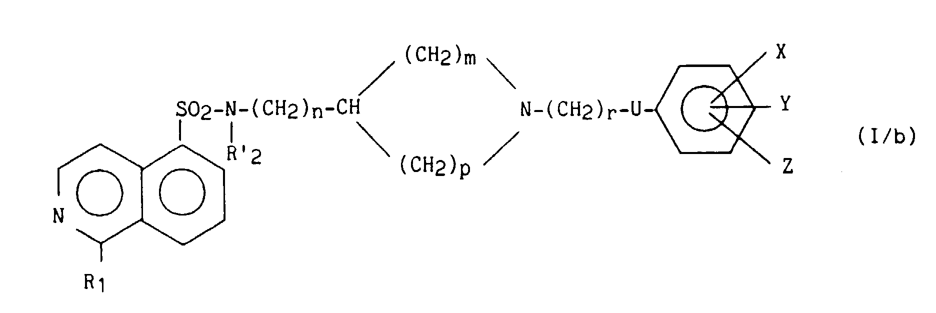 dans laquelle R₁, X, Y, Z, U, n, m, p, r et R'₂ sont tels que définis précédemment, cas particulier des composés de formule (I) où R₂ représente un groupement R'₂, l'ensemble des dérivés de formule (I/a) et (I/b) formant l'ensemble des dérivés de formule générale (I),composés de formule (I/a) et (I/b) dont on sépare, le cas échéant, les isomères, et que l'on salifie si on le désire avec un acide pharmaceutiquement acceptable,
composés de formule (I/a) et (I/b) que l'on purifie, si on le désire, par une technique de cristallisation et/ou de chromatographie.

10. Le procédé de préparation des composés de formule (I/c), partie des composés de formule (I) selon la revendication 1, 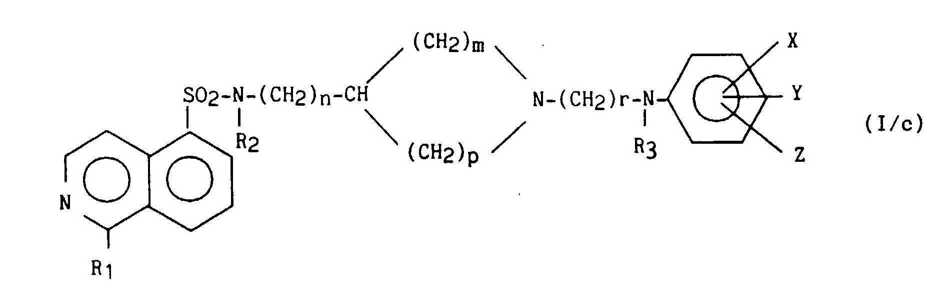 dans laquelle R₁, R₂, R₃, X, Y, Z, n, m, p et r ont la même signification que dans la formule (I),
caractérisé en ce que
- on condense une amine de formule (II/b) : 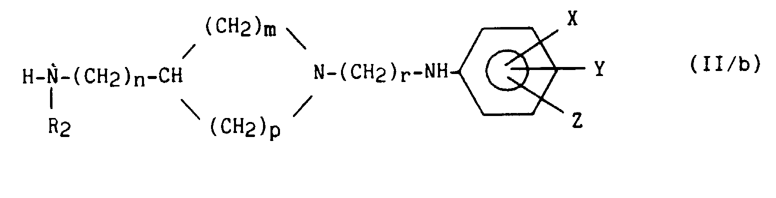 dans laquelle R₂, X, Y, Z, n, m, p et r sont tels que définis précedemment,
- avec un composé de formule (III), 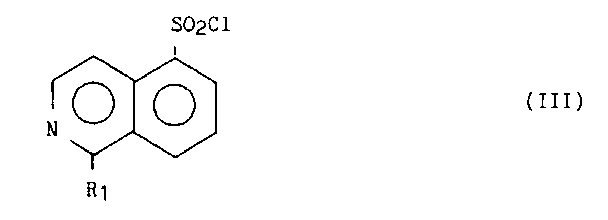 dans laquelle R₁ est tel que défini précédemment,
pour obtenir un composé de formule (I/d) : 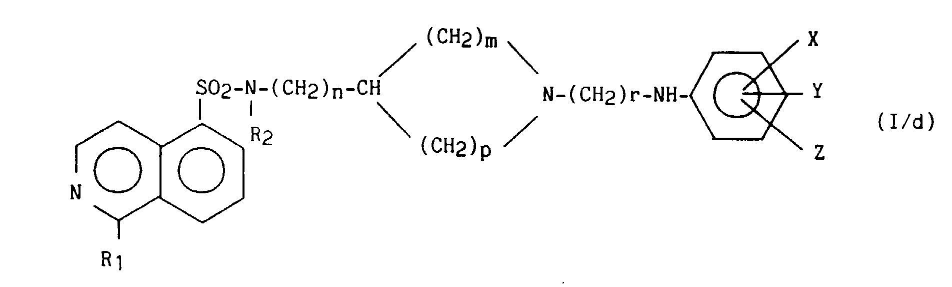 dans laquelle R₁, R₂, X, Y, Z, n, m, p et r sont tels que définis précédemment,
cas particulier des composés de formule (I/c) où R₃ représente un atome d'hydrogène,
composé de formule (I/d) que l'on substitue sur l'amine par un groupement de formule R'_{3,} où R'₃ a les mêmes significations que R₃ à l'exception de l'atome d'hydrogène, afin d'obtenir un composé de formule (I/e) : 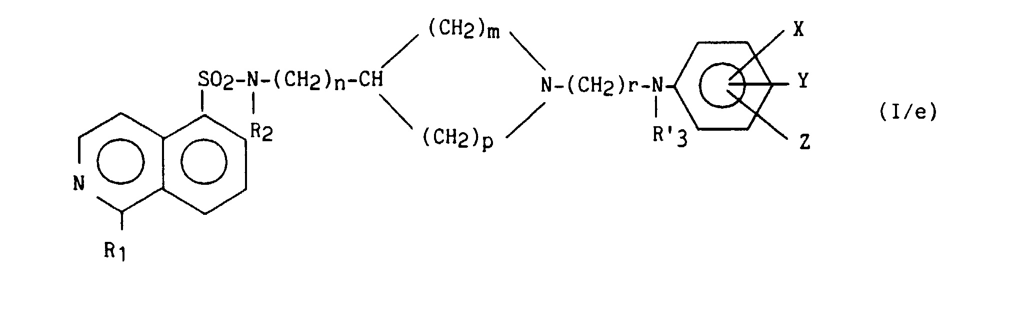 dans laquelle R₁, R₂, X, Y, Z, n, m, p, r et R'₃ sont tels que définis précédemment,
les composés de formule (I/d) et (I/e) formant l'ensemble des composés de formule (I/c),
composés de formule (I/c) dont on sépare, le cas échéant, les isomères, et que l'on salifie, si on le désire avec un acide pharmaceutiquement acceptable,
composés de formule (I/c) que l'on purifie, si on le désire, par une technique de cristallisation et/ou de chromatographie.

11. Composition pharmaceutique contenant comme principe actif au moins un composé selon les revendications 1 à 7 en combinaison avec un ou plusieurs excipients ou véhicules inertes, non toxiques, et pharmaceutiquement acceptables.

12. Composition pharmaceutique selon la revendication 11, utilisable dans le traitement et la prévention des affections dues ou reliées à des phénomènes de souffrance tissulaire et notamment dans le traitement et la prévention de l'ischémie myocardique et périphérique, des maladies vasculaires, de l'hypertension artérielle, de l'insuffisance cardiaque, des troubles du rythme cardiaque, des troubles liés au vieillissement artériel et à l'athérosclérose, des pathologies métaboliques constituant un facteur de risque cardiovasculaire, des thromboses, des affections relatives aux muscles lisses bronchiques, digestifs, urinaires, et utérins, des situations de souffrance tissulaire liées au vieillissement, à l'ischémie, à l'inflammation ou à une prolifération tissulaire y compris cancéreuse,
et également utilisable pour la prévention des resténoses ou thromboses vasculaires après pontage, dilatation vasculaire notamment coronaire, ou d'autres formes de reperméabilisation vasculaire.

## Revendications (Revendications pour l'(les) Etat(s) contractant(s) suivant(s): ES, GR)

1. Le procédé de préparation des composés de formule générale (I) : 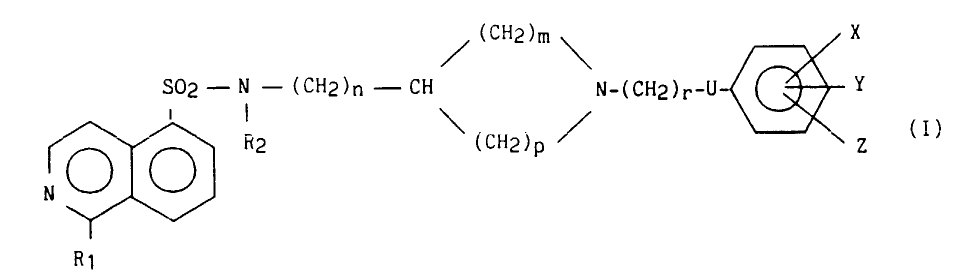 dans laquelle :
n est 0 ou un nombre entier de 1 à 4,
m et p sont des nombres entiers de 1 à 4, étant entendu que la somme m+p est 2,3,4 ou 5,
r est un nombre entier de 1 à 6,
R₁ représente un atome d'hydrogène, un atome de chlore ou un groupement hydroxy,
R₂ représente un atome d'hydrogène, un groupement alkyle inférieur, alkényl inférieur, alkynyle inférieur, cycloalkyle, cycloalkylalkyle inférieur, phényle ou phénylalkyle inférieur,
X, Y, Z, identiques ou différents, représentent un atome d'hydrogène, un atome d'halogène, ou un groupement choisi parmi alkyle inférieur, alcoxy inférieur, nitro, amino, cyano, acétamido, carboxamido, ou X et Y, ou Y et Z forment ensemble avec les 2 atomes de carbone du noyau phényle qui les portent, un cycle furanique, dihydrofuranique, ou benzénique,
U représente une liaison simple, un atome d'oxygène, un atome de soufre ou un groupement choisi parmi :
carbonyl, sulfinyl, sulfonyl, -NH-CO-, -CO-NH-, -O-(CH₂)r'-O- avec r' signifiant un nombre entier égal à 2 ou 3, -(CH₂)_{r"}-O-(CH₂)_{r"'}-
avec r" et r"' signifiant des nombres entiers égaux à 1 ou 2, et 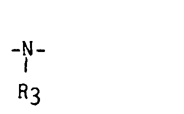 où
R₃ représente :
- un atome d'hydrogène,
- un groupement formyle,
- un groupement -A, -CO-A, ou -CO-O-A, avec A signifiant un groupement alkyle inférieur, alkényle inférieur, alkynyle inférieur, cycloalkyle, ou cycloalkylalkyle inférieur,
- un groupement -(CH₂)_{q}-phényl ou un groupement -(CH₂)_{q}-phényl substitué avec q signifiant 0 ou un nombre entier de 1 à 4,
- un groupement -CO-phényl ou un groupement -CO-phényl substitué,
- un groupement -CO-O-phényl ou un groupement -CO-O-phényl substitué,
- un groupement -CO-NR₄R₅
où R₄, R₅ :
- identiques ou différents, représentent un atome d'hydrogène ou un groupement choisi parmi alkyle inférieur, alkényl inférieur, alkynyl inférieur, phényle, phénylalkyle inférieur,
- soit forment avec l'atome d'azote qui les porte un cycle saturé de 4 à 7 chaînons,
[étant entendu que le terme "substitué" affectant les groupements -(CH₂)_{q}-phényl, -CO-phényl, ou -CO-O-phényl, signifie que ces groupements peuvent être substitués par un ou plusieurs radicaux choisis parmi : alkyle inférieur, alcoxy inférieur, hydroxy, atome d'halogène, et trifluorométhyle,
étant entendu
- que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkynyle inférieur" désignent des groupements insaturés linéaires ou ramifiés contenant de 2 à 6 atomes de carbone,
- que le terme "cycloalkyl" désigne un cycle carboné saturé contenant de 3 à 8 chaînons ;]
de leurs éventuels isomères optiques, ainsi que le cas échéant, de leurs sels d'addition à un acide pharmaceutiquement acceptable,
caractérisé en ce que :
a) on condense une amine de formule (II) : 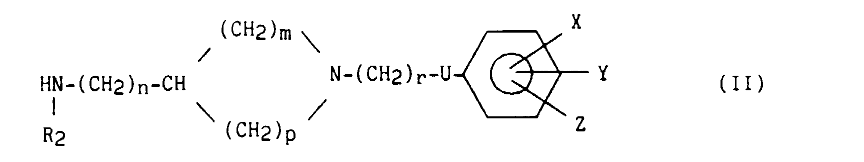 dans laquelle R₂, X, Y, Z, U, n, m, p, et r ont les mêmes significations que dans la formule générale (I),
avec un composé de formule (III) : 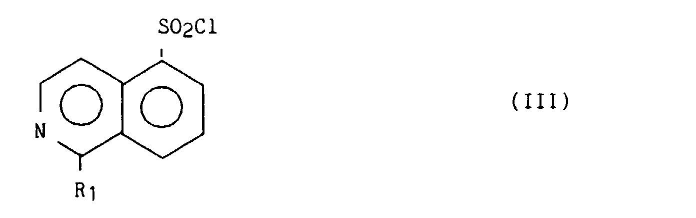 dans laquelle R₁ a la même signification que dans la formule générale (I),
pour obtenir un composé de formule (I),
dont on sépare, le cas échéant, les isomères, et que l'on salifie, si on le désire, avec un acide pharmaceutiquement acceptable, ou
b) on condense une amine de formule (II/a) : 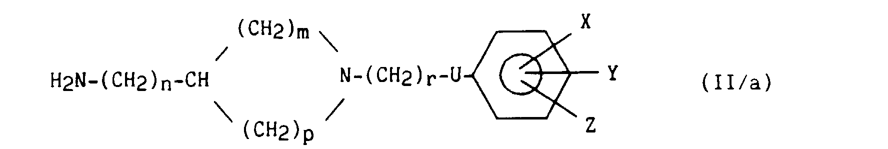 dans laquelle X, Y, Z, U, n, m, p, et r ont les mêmes significations que dans la formule générale (I),
avec un composé de formule (III) : 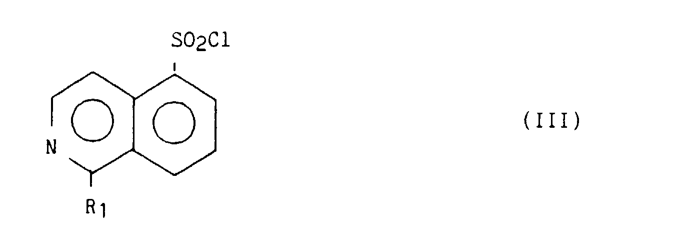 dans laquelle R₁ a la même signification que dans la formule générale (I),
pour obtenir un composé de formule (I/a) : 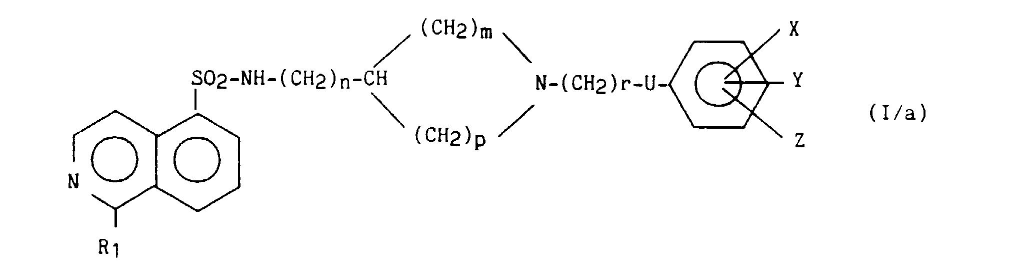 avec R₁, X, Y, Z, U, n, m, p et r tels que définis précédemment,
composé de formule (I/a) que l'on fait réagir avec un composé de formule R′₂-Hal avec R'₂ représentant un groupement alkyle inférieur, alkényle inférieur, alkynyle inférieur, cycloalkyle, cycloalkylalkyle inférieur, phényl ou phénylalkyle inférieur,
afin d'obtenir un composé de formule (I/b) : 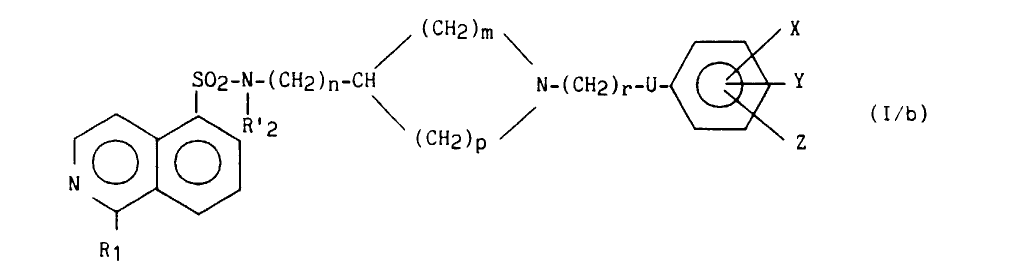 dans laquelle R₁, X, Y, Z, U, n, m, p, r et R'₂ sont tels que définis précédemment, l'ensemble des dérivés de formule (I/a) et (I/b) formant l'ensemble des dérivés de formule générale (I) et on sépare, le cas échéant, les isomères des composés de formule (I/a) et (I/b), et l'on salifie si on le désire avec un acide pharmaceutiquement acceptable.

2. Le procédé de préparation des composés (I) répondant plus précisément à la formulé I/c : 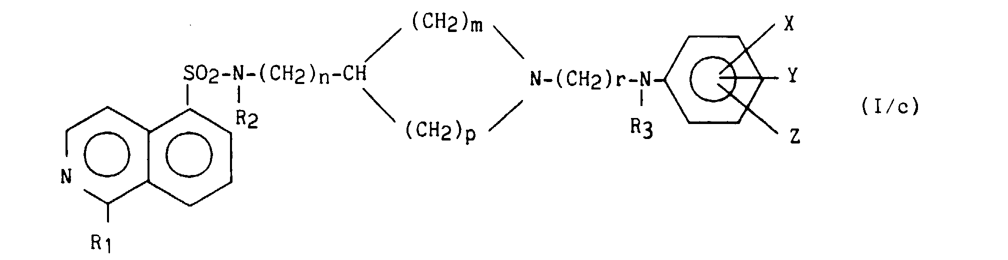 dans laquelle R₁, R₂, R₃, X, Y, Z, n, m, p et r ont les significations définies dans la revendication 1,
caractérisé en ce que
- on condense une amine de formule (II/b) : 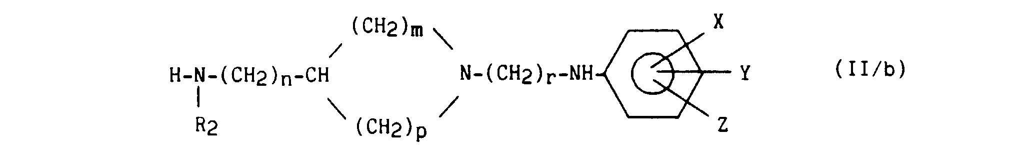 dans laquelle R₂, X, Y, Z, n, m, p et r sont tels que définis dans la revendication 1,
- avec un composé de formule (III), 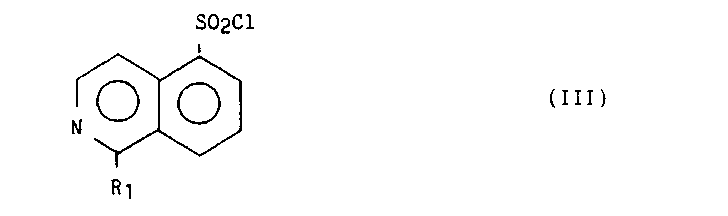 dans laquelle R₁ est tel que défini dans la revendication 1,
pour obtenir un composé de formule (I/d) : 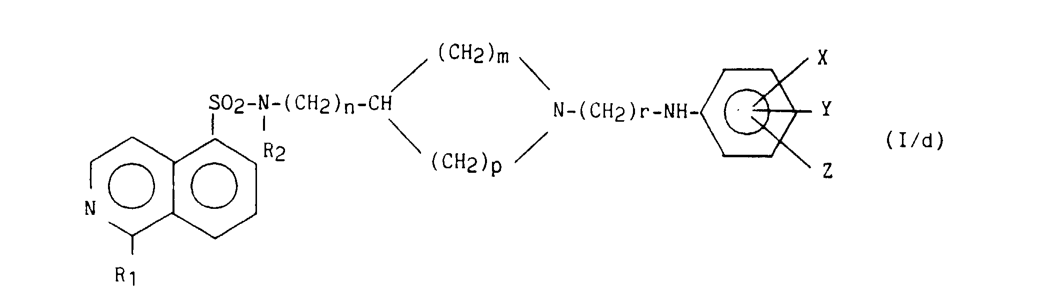 dans laquelle R₁, R2, X, Y, Z, n, m, p et r sont tels que définis précédemment,
lequel composé de formule (I/d) est substitué sur l'amine par un groupement de formule R'_{3,} où R'₃ a les mêmes significations que R₃ à l'exception de l'atome d'hydrogène, afin d'obtenir un composé de formule (I/e) : 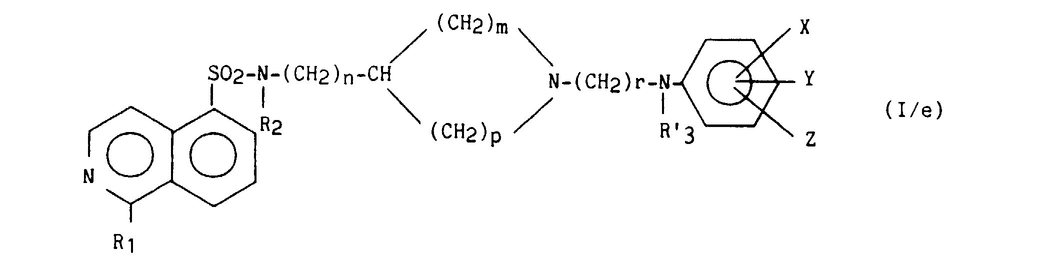 dans laquelle R₁, R₂, X, Y, Z, n, m, p, r et R'₃ sont tels que définis précédemment,
- composé de formule (I/c) dont on sépare, le cas échéant, les isomères, et que l'on salifie, si on le désire avec un acide pharmaceutiquement acceptable.

## Patentansprüche (Patentansprüche für folgende(n) Vertragsstaat(en): AT, BE, CH, DE, DK, FR, GB, IT, LI, LU, NL, PT, SE)

1. Verbindungen der allgemeinen Formel (I): 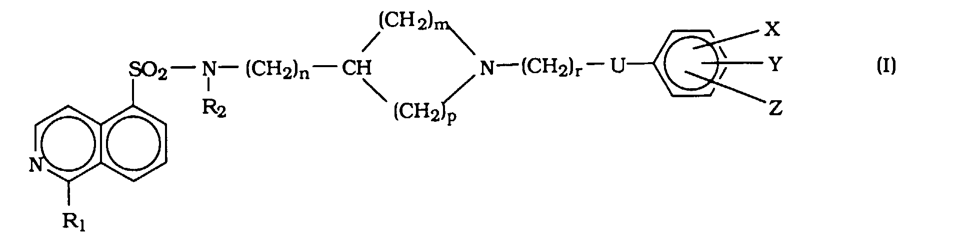 in der:
n 0 oder eine ganze Zahl mit einem Wert von 1 bis 4.
m und p ganze Zahlen mit einem Wert von 1 bis 4 mit der Maßgabe. daß die Summe von m + p 2. 3, 4 oder 5 beträgt.
r eine ganze Zahl mit einem Wert von 1 bis 6,
R₁ ein Wasserstoffatom, ein Chloratom oder eine Hydroxylgruppe.
R₂ ein Wasserstoffatom, eine Niedrigalkylgruppe, eine Niedrigalkenylgruppe, eine Niedrigalkinylgruppe, eine Cycloalkylgruppe, eine Niedrigcycloalkylalkylgruppe, eine Phenylgruppe oder eine Niedrigphenylalkylgruppe,
X, Y und Z, die gleichartig oder verschieden sein können, jeweils ein Wasserstoffatom, ein Halogenatom oder eine aus Niedrigalkylgruppen, Niedrigalkoxygruppen, Nitrogruppen, Aminogruppen, Cyanogruppen, Acetamidogruppen und Carboxamidogruppen ausgewählte Gruppe oder X und Y oder Y und Z gemeinsam mit den beiden sie tragenden Kohlenstoffatomen des Phenylkerns einen Furan-, Dihydrofuran- oder Benzolring und
U eine Einfachbindung, ein Sauerstoffatom, ein Schwefelatom oder eine Gruppe ausgewählt aus:
Carbonyl-, Sulfinyl, Sulfonyl, -NH-CO-, -CO-NH-, -O-(CH₂)_{r'}-O-, worin r' eine ganze Zahl mit einem Wert von 2 oder 3 darstellt, -(CH₂)_{r"}-O-(CH₂)_{r"'}-, worin r" und r"' ganze Zahlen mit Werten von 1 oder 2 darstellen und 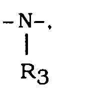 in der
R₃:
- ein Wasserstoffatom,
- eine Formylgruppe,
- eine Gruppe -A, -CO-A oder -CO-O-A, worin A eine Niedrigalkylgruppe, Niedrigalkenylgruppe, Niedrigalkinylgruppe, Cycloalkylgruppe oder Niedrigcycloalkylalkylgruppe.
- eine -(CH₂)_{q}-Phenylgruppe oder eine substituierte -(CH₂)_{q}-Phenylgruppe, worin q 0 oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe,
- eine Gruppe -CO-NR₄R₅,
in der R₄, R₅, die gleichartig oder verschieden sein können, ein Wasserstoffatom oder eine Gruppe ausgewählt aus Niedrigalkylgruppen, Niedrigalkenylgruppen, Niedrigalkinylgruppen, Phenylgruppen und Niedrigphenylalkylgruppen darstellen,
- oder zusammen mit dem sie tragenden Stickstoffatom einen gesättigten Ring mit 4 bis 7 Kettengliedern bilden,
wobei der Begriff "substituiert" bezüglich der -(CH₂)_{q}-Phenyl-, -CO-Phenyl- oder -CO-O-Phenylgruppen bedeutet, daß diese Gruppen durch einen oder mehrere Reste ausgewählt aus Niedrigalkylgruppen, Niedrigalkoxygruppen, Hydroxylgruppen, Halogenatomen und Trifluormethylgruppen substituiert sein können,
- die Begriffe "Niedrigalkyl" und "Niedrigalkoxy" für gesättigte, geradkettige oder verzweigte Kohlenstoffgruppen 1 bis 6 Kohlenstoffatome stehen,
- die Begriffe "Niedrigalkenyl" und "Niedrigalkinyl" für ungesättigte, geradkettige oder verzweigte Gruppen, die 2 bis 6 Kohlenstoffatome enthalten, stehen,
- der Begriff "Cycloalkyl" für einen gesättigten Kohlenstoffring mit 3 bis 8 Kohlenstoffatomen steht,
deren eventuelle optische Isomeren sowie gegebenenfalls deren Additionssalze mit einer pharmazeutisch annehmbaren Säure.

2. Verbindungen der Formel (I) nach Anspruch 1, in der m und p jeweils 2 bedeuten, sowie gegebenenfalls deren Additionssalze mit einer pharmazeutisch annehmbaren Säure.

3. Verbindung nach Anspruch 1, nämlich N-Methyl-N-{1-[4-(p-fluorphenoxy)-butyl]-piperid-4-yl}-(isochinolin-5-yl)-sulfonamid, sowie dessen Additionssalze mit einer pharmazeutisch annehmbaren Säure.

4. Verbindung nach Anspruch 1, nämlich N-Methyl-N-{[1-[3-(p-fluorphenoxy)-propyl]-piperid-4-yl)-methyl}-(isochinolin-5-yl)-sulfonamid, sowie dessen Additionssalze mit einer pharmazeutisch annehmbaren Säure.

5. Verbindung nach Anspruch 1, nämlich N-Methyl-N-{[1-[3-(p-fluorphenoxy)-propyl]-pyrrolidin-3-yl]-methyl}-(isochinolin-5-yl)-sulfonamid, dessen optische Isomeren sowie dessen Additionssalze mit einer pharmazeutisch annehmbaren Säure.

6. Verbindung nach Anspruch 1, nämlich N-{[1-[3-(p-Fluorphenylamino)-propyl]-piperid-4-yl]-methyl}-(isochinolin-5-yl)-sulfonamid, sowie dessen Additionssalze mit einer pharmazeutisch annehmbaren Säure.

7. Verbindung nach Anspruch 1, nämlich N-Methyl-N-{ [1-[4-(p-fluorphenyl)butyl]-piperid-4-yl)-methyl}-(isochinolin-5-yl)-sulfonamid, sowie dessen Additionssalze mit einer pharmazeutisch annehmbaren Säure.

8. Verfahren zur Herstellung der Verbindungen der Formel (I) nach Anspruch 1, **dadurch gekennzeichnet,** daß man:
- ein Amin der Formel (II): 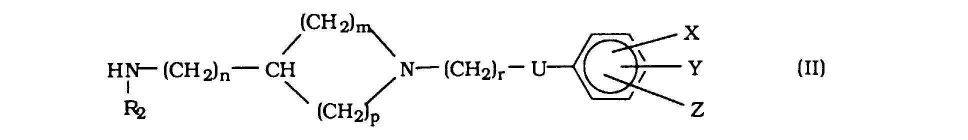 in der R₂, X, Y, Z, U, n, m, p und r die bezüglich der allgemeinen Formel (I) angegebenen Bedeutungen besitzen,
mit einer Verbindung der Formel (III): 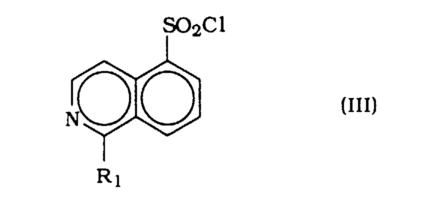 in der R₁ die bezüglich der Formel (I) angegebenen Bedeutungen besitzt, kondensiert zur Bildung einer Verbindung der Formel (I): 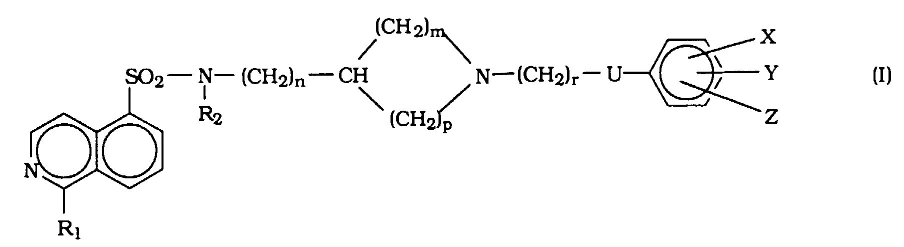 in der R₁, R₂, X, Y, Z, U, n, m, p und r die oben angegebenen Bedeutungen besitzen, die man gegebenenfalls in die Isomeren auftrennt und gewünschtenfalls mit einer pharmazeutisch annehmbaren Säure in ein Salz überführt oder welche Verbindungen der Formel (I) man gewünschtenfalls mit Hilfe einer Kristallisations- und/oder Chromatographie-Methode reinigt.

9. Verfahren zur Herstellung der Verbindungen der Formel (I) nach Anspruch 1, **dadurch gekennzeichnet,** daß man
- ein Amin der Formel (II/a): 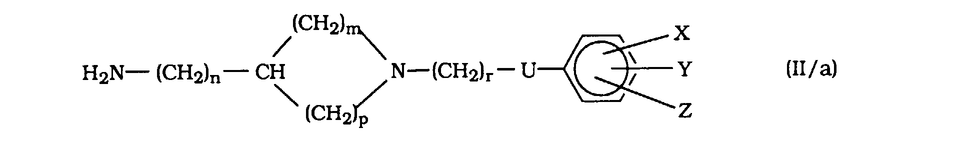 in der X, Y, Z, U, n, m, p und r die bezüglich der allgemeinen Formel (I) angegebenen Bedeutungen besitzen,
mit einer Verbindung der Formel (III): 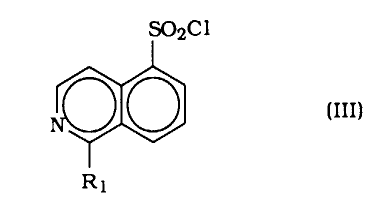 in der R₁ die bezüglich der allgemeinen Formel (I) angegebenen Bedeutungen besitzt, kondensiert zur Bildung einer Verbindung der Formel (I/a): 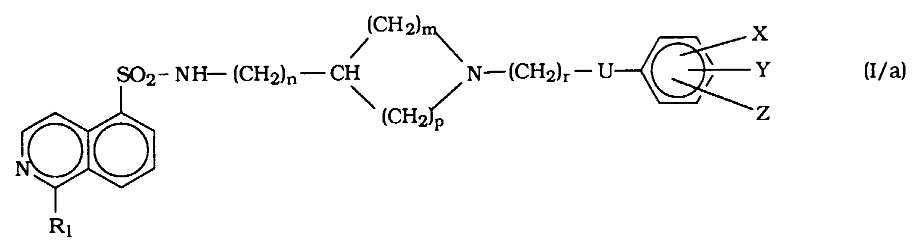 in der R₁, X, Y, Z, U, n, m, p und r die oben angegebenen Bedeutungen besitzen, einem Sonderfall der Verbindungen der Formel (I), in der R₂ ein Wasserstoffatom darstellt,
welche Verbindung der Formel (I/a) man mit einer Verbindung der Formel R'₂-Hal, in der R'₂ eine Niedrigalkylgruppe, Niedrigalkenylgruppe, Niedrigalkinylgruppe, Cycloalkylgruppe, Niedrigcycloalkylalkylgruppe, Phenylgruppe oder Niedrigphenylalkylgruppe darstellt, umsetzt,
zur Bildung einer Verbindung der Formel (I/b): 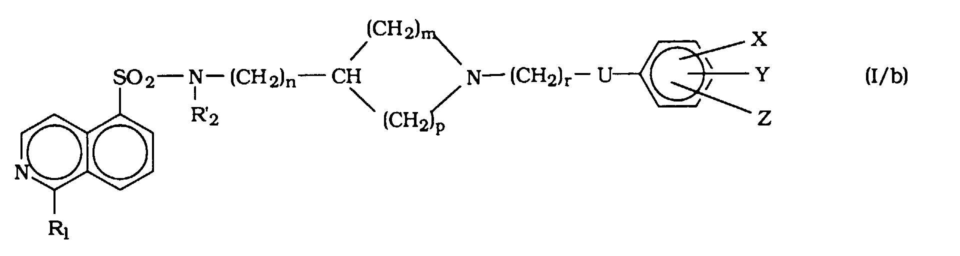 in der R₁, X, Y, Z, U, n, m, p, r und R'₂ die oben angegebenen Bedeutungen besitzen, einem Sonderfall der Verbindungen der Formel (I), in der R₂ eine Gruppe R'₂ darstellt, wobei die Gesamtheit der Derivate der Formeln (I/a) und (I/b) die Gesamtheit der Derivate der allgemeinen Formel (I) darstellt, welche Verbindungen der Formeln (I/a) und (I/b) man gegebenenfalls in die Isomeren auftrennt und gewünschtenfalls mit einer pharmazeutisch annehmbaren Säure in ein Salz überführt, und welche Verbindungen der Formeln (I/a) und (I/b) man gewünschtenfalls mit Hilfe einer Kristallisations- und/oder Chromatographie-Methode reinigt.

10. Verfahren zur Herstellung der Verbindungen der Formel (I/c), einem Teil der Verbindungen der Formel (I) nach Anspruch 1: 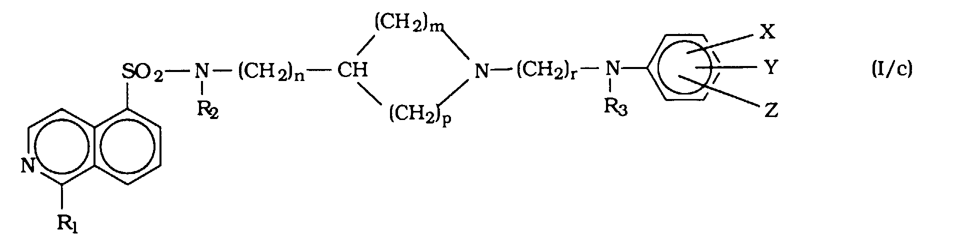 in der R₁, R₂, R₃, X, Y, Z, n, m, p und r die bezüglich der Formel (I) angegebenen Bedeutungen besitzen,
**dadurch gekennzeichnet,** daß man
- ein Amin der Formel (II/b): 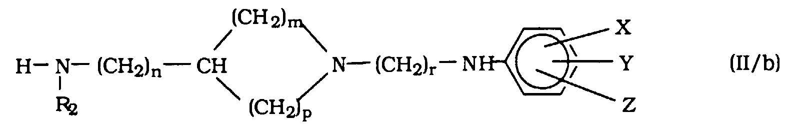 in der R₂, X, Y, Z, n, m, p und r die oben angegebenen Bedeutungen besitzen,
- mit einer Verbindung der Formel (III) 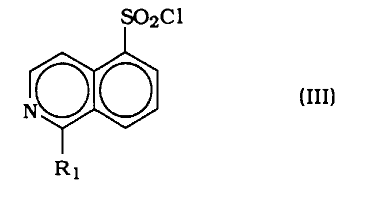 in der R₁ die oben angegebenen Bedeutungen besitzt,
kondensiert zur Bildung einer Verbindung der Formel (I/d): 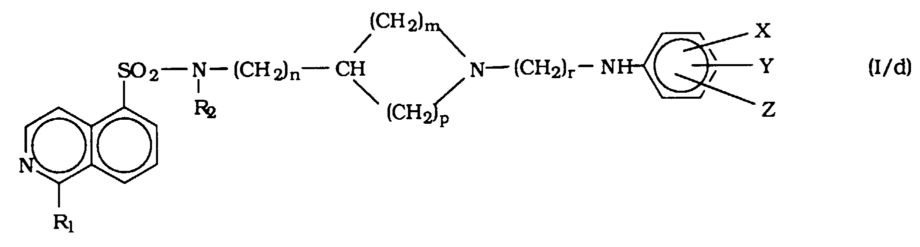 in der R₁, R₂, X, Y, Z, n, m, p und r die oben angegebenen Bedeutungen besitzen, einem Sonderfall der Verbindungen der Formel (I/c), worin R₃ ein Wasserstoffatom darstellt,
welche Verbindung der Formel (I/d) man am Amin mit einer Gruppe der Formel R'₃ substituiert, worin R'₃ die gleichen Bedeutungen wie R₃ besitzt mit Ausnahme des Wasserstoffatoms, so daß man eine Verbindung der Formel (I/e) erhält: 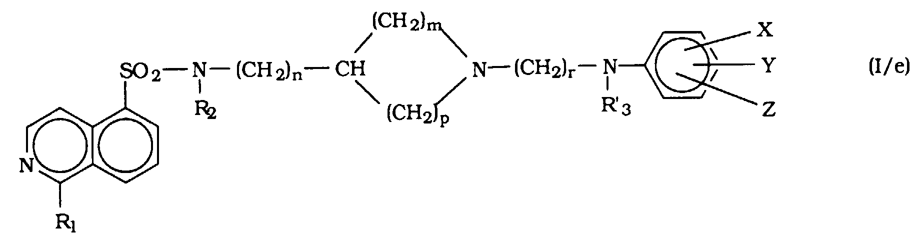 in der R₁, R₂, X, Y, Z, n, m, p, r und R'₃ die oben angegebenen Bedeutungen besitzen,
wobei die Verbindungen der Formeln (I/d) und (I/e) die Gesamtheit der Verbindungen der Formel (I/c) bilden,
welche Verbindungen der Formel (I/c) man gegebenenfalls in die Isomeren auftrennt und gewünschtenfalls mit einer pharmazeutisch annehmbaren Säure in die Salze überführt,
welche Verbindungen der Formel (I/c) man gewünschtenfalls mit einer Kristallisations- und/oder Chromatographie-Methode reinigt.

11. Pharmazeutische Zubereitung enthaltend als Wirkstoff mindestens eine Verbindung nach den Ansprüchen 1 bis 7 in Kombination mit einem oder mehreren inerten, nichttoxischen und pharmazeutisch annehmbaren Trägermaterialien oder Bindemitteln.

12. Pharmazeutische Zubereitung nach Anspruch 11 zur Verwendung bei der Behandlung und der Vorbeugung von Erkrankungen, die eine Folge von oder verknüpft sind mit Phänomenen von Gewebeerkrankungen und insbesondere bei der Behandlung und der Vorbeugung von Myokard- und peripherer Ischämie, von Gefäßerkrankungen, der arteriellen Hypertension, von Herzinsuffizienzen, von Störungen des Herzrhythmus, von mit dem Altern von Arterien und der Atherosklerose verknüpften Störungen, von Stoffwechselerkrankungen, die einen kardiovaskulären Risikofaktor darstellen, von Thrombosen, von Erkrankungen betreffend die glatten Muskeln der Bronchien, des Verdauungstrakts, des Harntrakts und des Uterus, von Situationen von Gewebeerkrankungen, die mit dem Altern, der Ischämie, der Entzündung oder einer Zellwucherung einschließlich einer krebsartigen verknüpft sind,
und die auch zur Vorbeugung von Gefäßrestenosen oder -thrombosen nach Bypass-Operationen, der Gefäßerweiterung, insbesondere von Koronargefäßen, oder von anderen Formen der Durchgängigmachung von Gefäßen verwendbar sind.

## Patentansprüche (Patentansprüche für folgende(n) Vertragsstaat(en): ES, GR)

1. Verfahren zur Herstellung der Verbindungen der allgemeinen Formel (I): 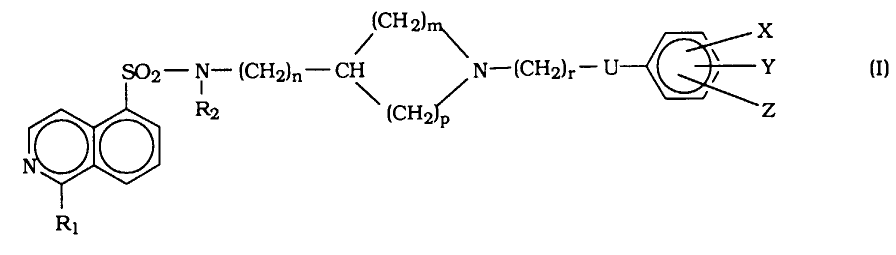 in der:
n 0 oder eine ganze Zahl mit einem Wert von 1 bis 4,
m und p ganze Zahlen mit einem Wert von 1 bis 4 mit der Maßgabe,
daß die Summe von m + p 2, 3, 4 oder 5 beträgt,
r eine ganze Zahl mit einem Wert von 1 bis 6,
R₁ ein Wasserstoffatom, ein Chloratom oder eine Hydroxylgruppe,
R₂ ein Wasserstoffatom, eine Niedrigalkylgruppe, eine Niedrigalkenylgruppe, eine Niedrigalkinylgruppe, eine Cycloalkylgruppe, eine Niedrigcycloalkylalkylgruppe, eine Phenylgruppe oder eine Niedrigphenylalkylgruppe,
X, Y und Z, die gleichartig oder verschieden sein können, jeweils ein Wasserstoffatom, ein Halogenatom oder eine aus Niedrigalkylgruppen, Niedrigalkoxygruppen, Nitrogruppen, Aminogruppen, Cyanogruppen, Acetamidogruppen und Carboxamidogruppen ausgewählte Gruppe oder X und Y oder Y und Z gemeinsam mit den beiden sie tragenden Kohlenstoffatomen des Phenylkerns einen Furan-, Dihydrofuran- oder Benzolring und
U eine Einfachbindung, ein Sauerstoffatom, ein Schwefelatom oder eine Gruppe ausgewählt aus:
Carbonyl-, Sulfinyl, Sulfonyl, -NH-CO-, -CO-NH-, -O-(CH₂)_{r'}-O-, worin r' eine ganze Zahl mit einem Wert von 2 oder 3 darstellt, -(CH₂)_{r"}-O-(CH₂)_{r"'}-, worin r"
und r"' ganze Zahlen mit Werten von 1 oder 2 darstellen und 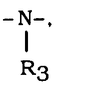 in der
R₃:
- ein Wasserstoffatom,
- eine Formylgruppe,
- eine Gruppe -A, -CO-A oder -CO-O-A, worin A eine Niedrigalkylgruppe, Niedrigalkenylgruppe, Niedrigalkinylgruppe, Cycloalkylgruppe oder Niedrigcycloalkylalkylgruppe,
- eine -(CH₂)_{q}-Phenylgruppe oder eine substituierte -(CH₂)_{q}-Phenylgruppe, worin q 0 oder eine ganze Zahl mit einem Wert von 1 bis 4 darstellt,
- eine -CO-Phenylgruppe oder eine substituierte -CO-Phenylgruppe,
- eine -CO-O-Phenylgruppe oder eine substituierte -CO-O-Phenylgruppe,
- eine Gruppe-CO-NR₄R₅,
in der R₄, R₅, die gleichartig oder verschieden sein können, ein Wasserstoffatom oder eine Gruppe ausgewählt aus Niedrigalkylgruppen, Niedrigalkenylgruppen, Niedrigalkinylgruppen, Phenylgruppen und Niedrigphenylalkylgruppen darstellen,
- oder zusammen mit dem sie tragenden Stickstoffatom einen gesättigten Ring mit 4 bis 7 Kettengliedern bilden,
[wobei der Begriff "substituiert" bezüglich der -(CH₂)_{q}-Phenyl-, -CO-Phenyl- oder -CO-O-Phenylgruppen bedeutet, daß diese Gruppen durch einen oder mehrere Reste ausgewählt aus Niedrigalkylgruppen, Niedrigalkoxygruppen, Hydroxylgruppen, Halogenatomen und Trifluormethylgruppen substituiert sein können,
- die Begriffe "Niedrigalkyl" und "Niedrigalkoxy" für gesättigte, geradkettige oder verzweigte Kohlenstoffgruppen 1 bis 6 Kohlenstoffatome stehen,
- die Begriffe "Niedrigalkenyl" und "Niedrigalkinyl" für ungesättigte, geradkettige oder verzweigte Gruppen, die 2 bis 6 Kohlenstoffatome enthalten, stehen,
- der Begriff "Cycloalkyl" für einen gesättigten Kohlenstoffring mit 3 bis 8 Kohlenstoffatomen steht,]
sowie von deren eventuellen optischen Isomeren und gegebenenfalls von deren Additionssalzen mit einer pharmazeutisch annehmbaren Säure,
**dadurch gekennzeichnet**, daß man
a) ein Amin der Formel (II): 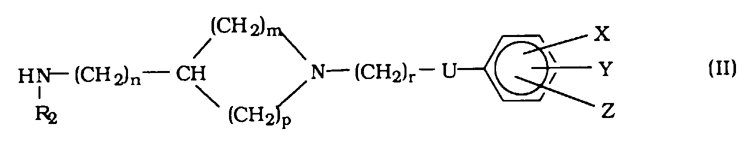 in der R₂, X, Y, Z, U, n, m, p und r die bezüglich der allgemeinen Formel (I) angegebenen Bedeutungen besitzen,
mit einer Verbindung der Formel (III): 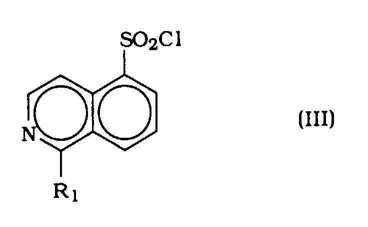 in der R₁ die bezüglich der Formel (I) angegebenen Bedeutungen besitzt, kondensiert zur Bildung einer Verbindung der Formel (I),
die man gegebenenfalls in die Isomeren auftrennt und gewünschtenfalls mit einer pharmazeutisch annehmbaren Säure in das Salz überführt, oder
b) ein Amin der Formel (II/a): 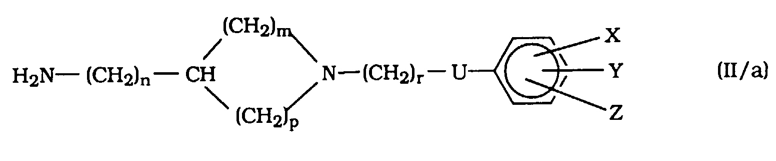 in der X, Y, Z, U, n, m, p und r die bezüglich der allgemeinen Formel (I) angegebenen Bedeutungen besitzen,
mit einer Verbindung der Formel (III): 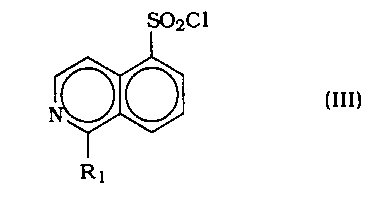 in der R₁ die bezüglich der allgemeinen Formel (I) angegebenen Bedeutungen besitzt,
kondensiert zur Bildung einer Verbindung der Formel (I/a): 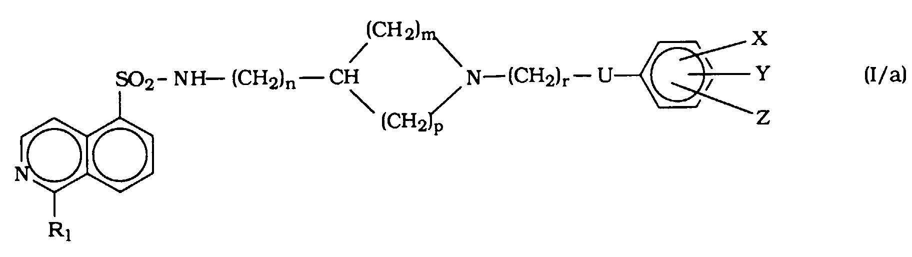 in der R₁, X, Y, Z, U, n, m, p und r die oben angegebenen Bedeutungen besitzen, welche Verbindung der Formel (I/a) man mit einer Verbindung der Formel R'₂-Hal, in der R'₂ eine Niedrigalkylgruppe, Niedrigalkenylgruppe, Niedrigalkinylgruppe, Cycloalkylgruppe, Niedrigcycloalkylalkylgruppe, Phenylgruppe oder Niedrigphenylalkylgruppe darstellt, umsetzt,
zur Bildung einer Verbindung der Formel (I/b): 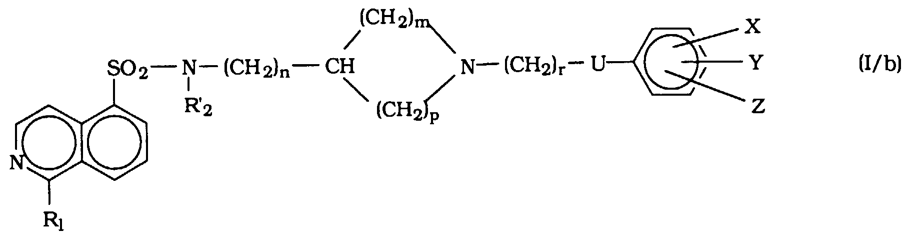 in der R₁, X, Y, Z, U, n, m, p, r und R'₂ die oben angegebenen Bedeutungen besitzen, wobei die Gesamtheit der Derivate der Formeln (I/a) und (I /b) die Gesamtheit der Derivate der allgemeinen Formel (I) darstellt, welche Verbindungen der Formeln (I/a) und (I/b) man gegebenenfalls in die Isomeren auftrennt und gewünschtenfalls mit einer pharmazeutisch annehmbaren Säure in ein Salz überführt.

2. Verfahren zur Herstellung der Verbindungen (I), die insbesondere der Formel (I/c) entsprechen: 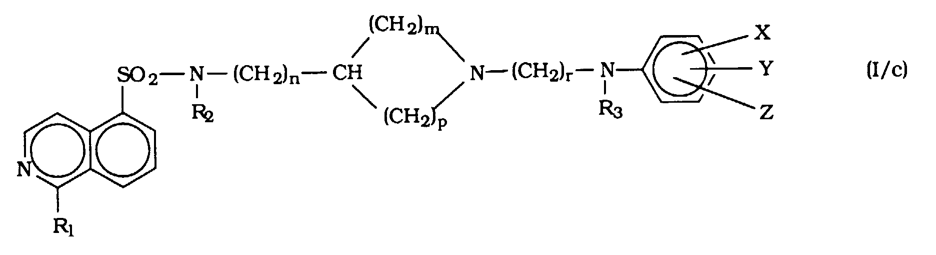 in der R₁, R₂, R₃, X, Y, Z, n, m, p und r die in Anspruch 1 angegebenen Bedeutungen besitzen,
**dadurch gekennzeichnet,** daß man
- ein Amin der Formel (II/b): 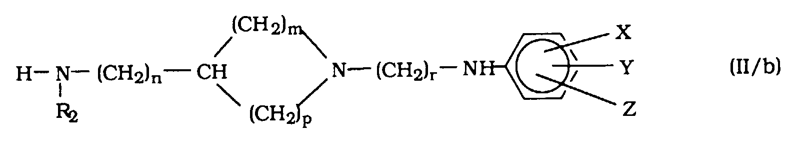 in der R₂, X, Y, Z, n, m, p und r die in Anspruch 1 angegebenen Bedeutungen besitzen,
- mit einer Verbindung der Formel (III) 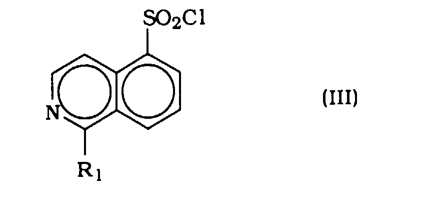 in der R₁ die in Anspruch 1 angegebenen Bedeutungen besitzt,
kondensiert zur Bildung einer Verbindung der Formel (I/d): 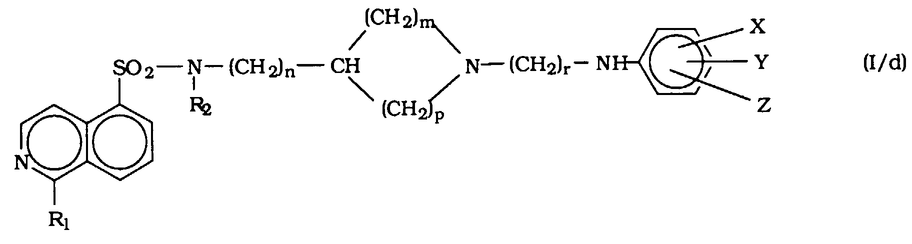 in der R₁, R₂, X, Y, Z, n, m, p und r die oben angegebenen Bedeutungen besitzen, welche Verbindung der Formel (I/d) man am Amin mit einer Gruppe der Formel R'₃ substituiert, worin R'₃ die gleichen Bedeutungen wie R₃ besitzt mit Ausnahme des Wasserstoffatoms, so daß man eine Verbindung der Formel (I/e) erhält: 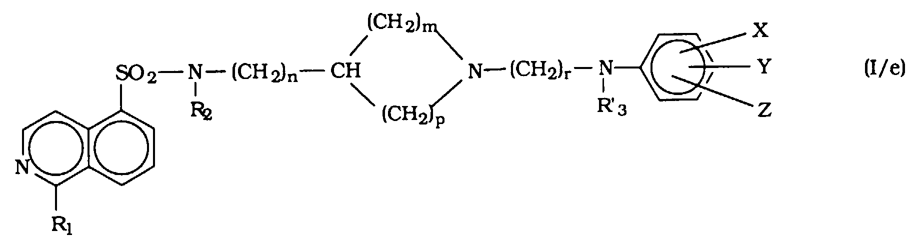 in der R₁, R₂, X, Y, Z, n, m, p, r und R'₃ die oben angegebenen Bedeutungen besitzen,
- welche Verbindung der Formel (I/c) man gegebenenfalls in die Isomeren auftrennt und gewünschtenfalls mit einer pharmazeutisch annehmbaren Säure in ein Salz überführt.

## Claims (Claims for the following Contracting State(s): AT, BE, CH, DE, DK, FR, GB, IT, LI, LU, NL, PT, SE)

1. Compounds of the general formula (I): 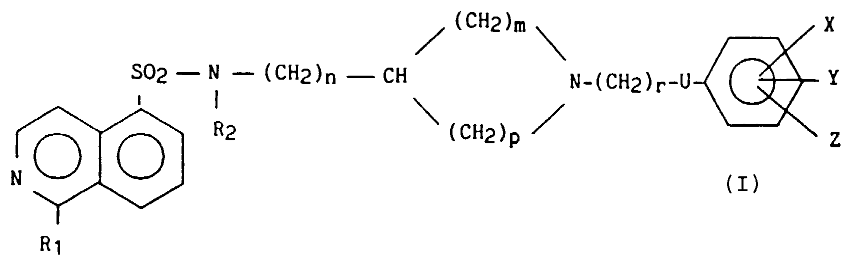 wherein :
m is 0 or an integer of from 1 to 4,
m and p are integers of from 1 to 4, it being understood that the sum m+p is 2, 3, 4 or 5,
r is an integer of from 1 to 6,
R₁ represents a hydrogen atom, a chlorine atom or a hydroxy group,
R₂ represents a hydrogen atom, or a lower alkyl, lower alkenyl, lower alkynyl, cycloalkyl, cycloalkyl-lower alkyl, phenyl or phenyl-lower alkyl group,
X, Y and Z, which are identical or different, each represents a hydrogen atom, a halogen atom, or a group selected from lower alkyl, lower alkoxy, nitro, amino, cyano, acetamido and carboxamido, or X and Y, or Y and Z, form together with the 2 carbon atoms of the phenyl nucleus that carry them, a furan, dihydrofuran or benzene ring,
U represents a single bond, an oxygen atom, a sulphur atom or a group selected from :
carbonyl, sulphinyl, sulphonyl, -NH-CO-, -CO-NH-, -O-(CH₂)_{r'}-O- wherein r' denotes an integer 2 or 3, -(CH₂)r_{"}-O-(CH₂)r_{"'}- wherein r" and r"' denote integers 1 or 2, and 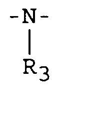 wherein
R₃ represents :
- a hydrogen atom,
- a formyl group,
- a group -A, -CO-A or -CO-O-A wherein A represents a lower alkyl, lower alkenyl, lower alkynyl, cycloalkyl or cycloalkyl-lower alkyl group,
- a -(CH₂)_{q}-phenyl group or a substituted
- (CH₂)_{q}-phenyl group wherein q represents 0 or an integer of from 1 to 4,
- a -CO-phenyl group or a substituted -CO-phenyl group,
- a -CO-O-phenyl group or a substituted -CO-O-phenyl group,
- a -CO-NR₄R₅ group
wherein R_{4'} R₅:
- identical or different, each represents a hydrogen atom or a group selected from lower alkyl, lower alkenyl, lower alkynyl, phenyl and phenyl-lower alkyl,
- or form with the nitrogen atom carrying them a saturated ring having from 4 to 7 chain members.
it being understood that the term "substituted" referring to the groups -(CH₂)_{q}-phenyl, -CO-phenyl or -CO-O-phenyl denotes that those groups may be substituted by one or more radicals selected from lower alkyl, lower alkoxy, hydroxy, halogen atom and trifluoromethyl,
it being understood that :
- the terms "lower alkyl" and "lower alkoxy" denote saturated straight-chain or branched carbon-containing groups containing from 1 to 6 carbon atoms,
- the terms "lower alkenyl" and "lower alkynyl" denote unsaturated straight-chain or branched groups containing from 2 to 6 carbon atoms,
- the term "cycloalkyl" denotes a saturated carbon ring containing from 3 to 8 chain members,
their possible optical isomers, and also, where appropriate, their addition salts with a pharmaceutically acceptable acid.

2. The compounds of formula (I) according to claim 1, wherein m and p each represents 2,
and also, where appropriate, their addition salts with a pharmaceutically acceptable acid.

3. The compound according to claim 1 that is N-methyl-N-{1-[4-(p-fluorophenoxy)butyl]piperid-4-yl}-(isoquinolin-5-yl)sulphonamide,
and also the addition salts thereof with a pharmaceutically acceptable acid.

4. The compound according to claim 1 that is N-methyl-N-{[1-[3-(p-fluorophenoxy)propyl]piperid-4-yl]methyl}-(isoquinolin-5-yl)sulphonamide,
and also the addition salts thereof with a pharmaceutically acceptable acid.

5. The compound according to claim 1 that is N-methyl-N-{[1-[3-(p-fluorophenoxy)propyl]pyrrolidin-3-yl]methyl}(isoquinolin-5-yl)sulphonamide,
the optical isomers thereof and also the addition salts thereof with a pharmaceutically acceptable acid.

6. The compound according to claim 1 that is N-{[1-[3-(p-fluorophenylamino)propyl]piperid-4-yl]methyl}-(isoquinolin-5-yl)sulphonamide,
and also the addition salts thereof with a pharmaceutically acceptable acid.

7. The compound according to claim 1 that is N-methyl-N-{[1-[4-(p-fluorophenyl)butyl]piperid-4-yl]methyl}(isoquinolin-5-yl)sulphonamide,
and also the addition salts thereof with a pharmaceutically acceptable acid.

8. A process for the preparation of compounds of formula (I) according to claim 1, characterised in that :
- an amine of formula (II) : 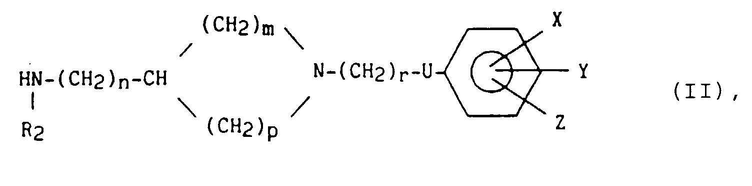 wherein R₂, X, Y, Z, U, n, m, p and r are as defined for the general formula (I), is condensed
with a compound of formula (III) : 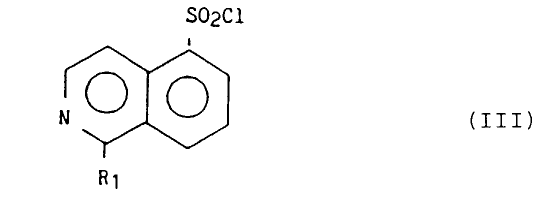 wherein R₁ is as defined for the general formula (I), to obtain a compound of formula (I) : 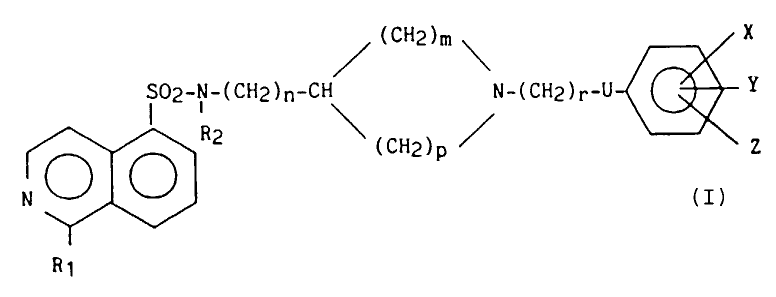 wherein R₁, R₂, X, Y, Z, U, n, m, p and r are as defined above, which, where appropriate, is separated into its isomers and which, if desired, is converted into a salt with a pharmaceutically acceptable acid, which compounds of formula (I) are purified, if desired, by a crystallisation and/or chromatography technique.

9. A process for the preparation of compounds of formula (I) according to claim 1, characterised in that :
- an amine of formula (II/a) : 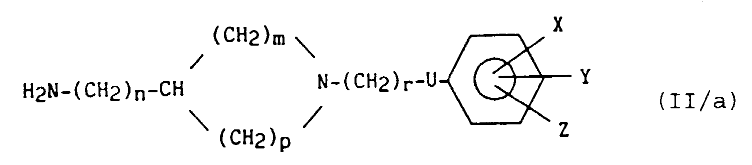 wherein X, Y, Z, U, n, m, p and r are as defined for the general formula (I), is condensed
with a compound of formula (III) : 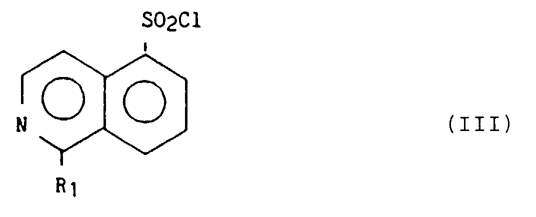 wherein R₁ is as defined for the general formula (I),
to obtain a compound of formula (I/a) : 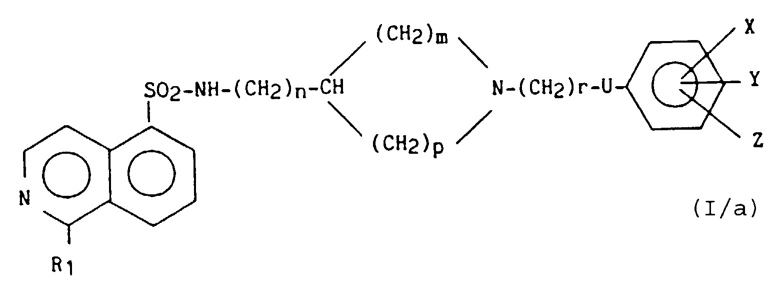 wherein R₁, X, Y, Z, U, n, m, p and r are as defined above, a particular case of compounds of formula (I) in which R₂ represents a hydrogen atom,
which compound of formula (I/a) is reacted with a compound of formula R'₂Hal, in which R'₂ represents a lower alkyl, lower alkenyl, lower alkynyl, cycloalkyl, cycloalkyl-lower alkyl, phenyl or phenyl-lower alkyl group,
to obtain a compound of formula (I/b) : 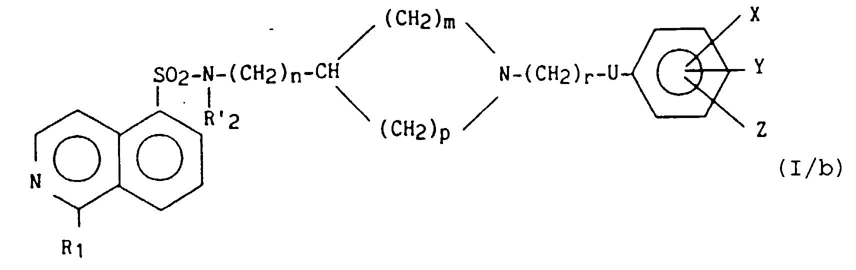 wherein R₁, X, Y, Z, U, n, m, p, r and R'₂ are as defined above, a particular case of compounds of formula (I) in which R₂ represents a R'₂ group, the totality of the compounds of formulae (I/a) and (I/b) forming the totality of the compounds of the general formula (I),
which compounds of formulae (I/a) and (I/b) are separated, where appropriate, into their isomers and converted into salts, if desired, with a pharmaceutically acceptable acid,
which compounds of formulae (I/a) and (I/b) are purified, if desired, by a crystallisation and/or chromatography technique.

10. A process for the preparation of compounds of formula (I/c), part of the compounds of formula (I) according to claim 1, 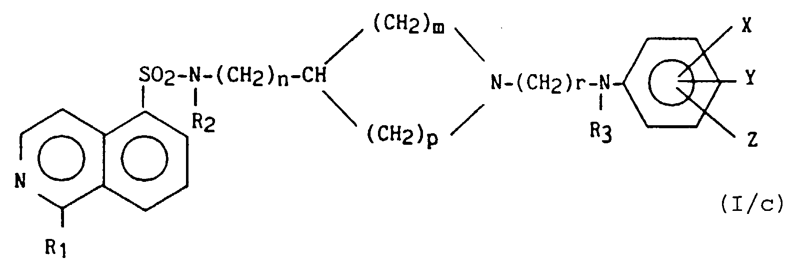 wherein R₁, R₂, R₃, X, Y, Z, n, m, p and r are as defined for formula (I),
characterised in that
- an amine of formula (II/b) : 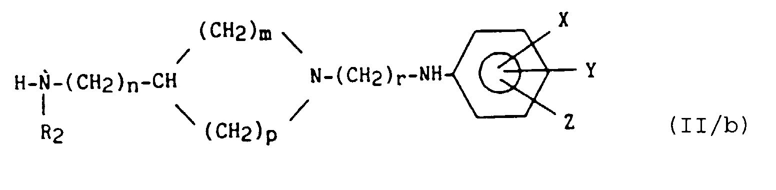 wherein R₂, X, Y, Z, n, m, p and r are as defined above, is condensed
- with a compound of formula (III) 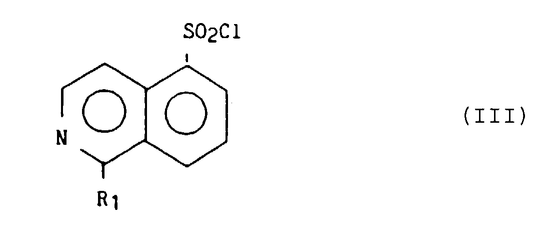 wherein R₁ is as defined above,
to obtain a compound of formula (I/d) : 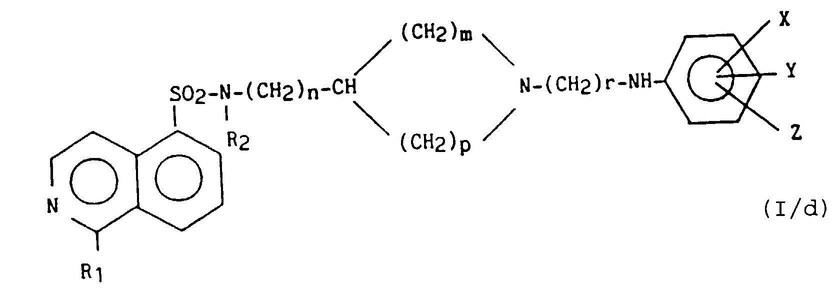 wherein R₁, R₂, X, Y, Z, n, m, p and r are as defined above,
a particular case of compounds of formula (I/c) in which R₃ represents a hydrogen atom,
which compound of formula (I/d) is substituted on the amine by a group of formula R'₃, wherein R'₃ has the same meanings as R₃ with the exception of the hydrogen atom,
to obtain a compound of formula (I/e) : 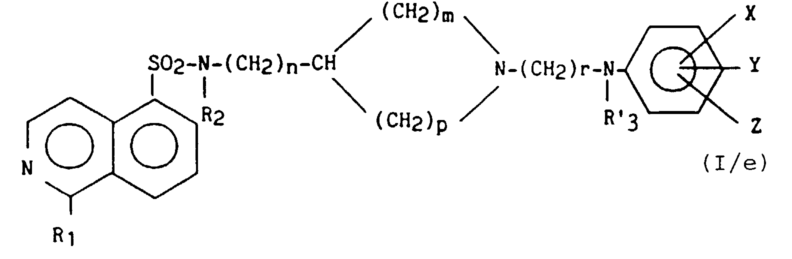 wherein R₁, R₂, X, Y, Z, n, m, p, r and R'₃ are as defined above,
the compounds of formulae (I/d) and (I/e) forming the totality of the compounds of formula (I/c),
which compounds of formula (I/c) are separated, where appropriate, into their isomers, and converted into salts, if desired, with a pharmaceutically acceptable acid,
which compounds of formula (I/c) are purified, if desired, by a crystallisation and/or chromatography technique.

11. Pharmaceutical composition comprising as active ingredient at least one compound according to claims 1 to 7, in combination with one or more pharmaceutically acceptable, inert, non-toxic excipients or carriers.

12. Pharmaceutical composition according to claim 11 that can be used in the treatment and prevention of conditions resulting from or associated with tissue damage phenomena, and especially in the treatment and prevention of myocardial and peripheral ischaemia, vascular disorders, arterial hypertension, cardiac insufficiency, cardiac rhythm disorders, disorders associated with arterial ageing and with atherosclerosis, metabolic pathologies constituting a cardiovascular risk factor, thromboses, conditions relating to bronchial, digestive, urinary and uterine smooth muscles, conditions of tissue damage associated with ageing, with ischaemia, with inflammation or with tissue proliferation, including cancerous proliferation,
and also that can be used in the prevention of vascular restenoses or thromboses after by-pass, vascular dilatation, especially coronary vascular dilatation, or other forms of vascular reopening.

## Claims (Claims for the following Contracting State(s): ES, GR)

1. A process for the preparation of compounds of the general formula (I) 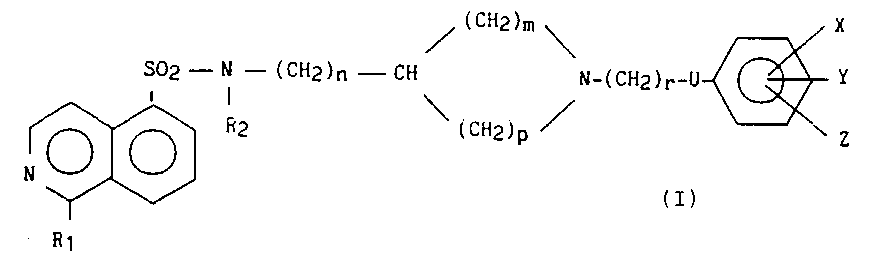 wherein :
m is 0 or an integer of from 1 to 4,
m and p are integers of from 1 to 4, it being understood that the sum m+p is 2, 3, 4 or 5,
r is an integer of from 1 to 6,
R₁ represents a hydrogen atom, a chlorine atom or a hydroxy group,
R₂ represents a hydrogen atom, or a lower alkyl, lower alkenyl, lower alkynyl, cycloalkyl, cycloalkyl-lower alkyl, phenyl or phenyl-lower alkyl group,
X, Y and Z, which are identical or different, each represents a hydrogen atom, a halogen atom, or a group selected from lower alkyl, lower alkoxy, nitro, amino, cyano, acetamido and carboxamido, or X and Y, or Y and Z, form together with the 2 carbon atoms of the phenyl nucleus that carry them, a furan, dihydrofuran or benzene ring,
U represents a single bond, an oxygen atom, a sulphur atom or a group selected from :
carbonyl, sulphinyl, sulphonyl, -NH-CO-, -CO-NH-, -O-(CH₂)_{r'}-O- wherein r' denotes an integer 2 or 3, -(CH₂)_{r"}-O-(CH₂)_{r"'}- wherein r" and r"' denote integers 1 or 2, and 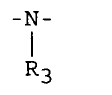 wherein
R₃ represents :
- a hydrogen atom,
- a formyl group,
- a group -A, -CO-A or -CO-O-A wherein A represents a lower alkyl, lower alkenyl, lower alkynyl, cycloalkyl or cycloalkyl-lower alkyl group,
- a -(CH₂)_{q}-phenyl group or a substituted
- (CH₂)_{q}-phenyl group wherein q represents 0 or an integer of from 1 to 4,
- a -CO-phenyl group or a substituted -CO-phenyl group,
- a -CO-O-phenyl group or a substituted -CO-O-phenyl group,
- a -CO-NR₄R₅ group
wherein R₄, R₅:
- identical or different, each represents a hydrogen atom or a group selected from lower alkyl, lower alkenyl, lower alkynyl, phenyl and phenyl-lower alkyl,
- or form with the nitrogen atom carrying them a saturated ring having from 4 to 7 chain members.
[it being understood that the term "substituted" referring to the groups -(CH₂)_{q}-phenyl, -CO-phenyl or -CO-O-phenyl denotes that those groups may be substituted by one or more radicals selected from lower alkyl, lower alkoxy, hydroxy, halogen atom and trifluoromethyl,
it being understood that :
- the terms "lower alkyl" and "lower alkoxy" denote saturated straight-chain or branched carbon-containing groups containing from 1 to 6 carbon atoms,
- the terms "lower alkenyl" and "lower alkynyl" denote unsaturated straight-chain or branched groups containing from 2 to 6 carbon atoms,
- the term "cycloalkyl" denotes a saturated carbon ring containing from 3 to 8 chain members],
their possible optical isomers, and also, where appropriate, their addition salts with a pharmaceutically acceptable acid,
characterised in that :
a) an amine of formula (II) : 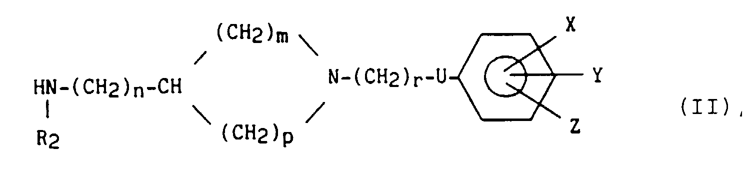 wherein R₂, X, Y, Z, U, n, m, p and r are as defined for the general formula (I), is condensed with a compound of formula (III) : 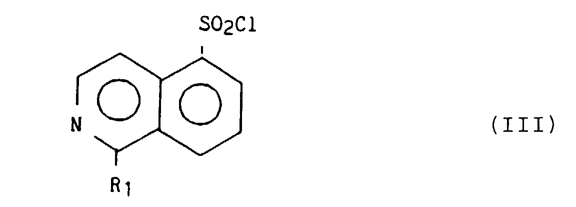 wherein R₁ is as defined for the general formula (I),
to obtain a compound of formula (I),
which, where appropriate, is separated into its isomers and which, if desired, is converted into a salt with a pharmaceutically acceptable acid, or
b) an amine of formula (IIIa) : 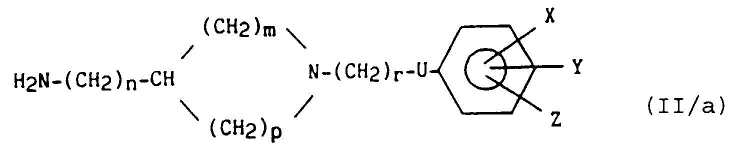 wherein X, Y, Z, U, n, m, p and r are as defined for the general formula (I), is condensed
with a compound of formula (III) : 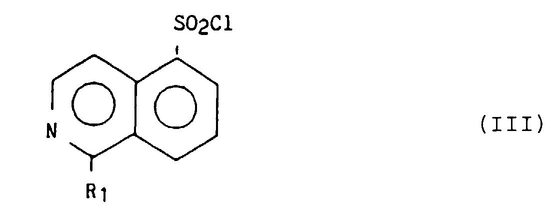 wherein R₁ is as defined for the general formula (I),
to obtain a compound of formula (I/a) 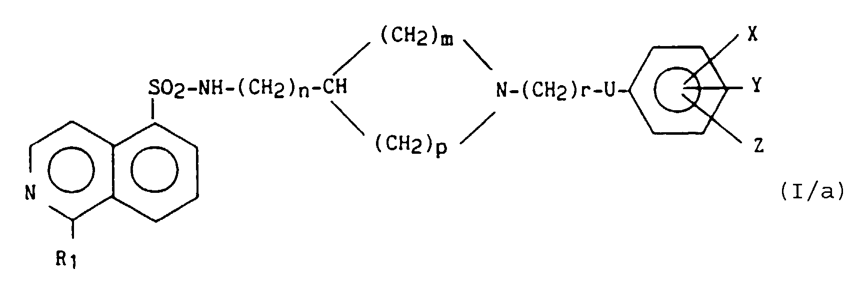 wherein R₁, X, Y, Z, U, n, m, p and r are as defined above,
which compound of formula (I/a) is reacted with a compound of formula R'₂Hal, in which R'₂ represents a lower alkyl, lower alkenyl, lower alkynyl, cycloalkyl, cycloalkyl-lower alkyl, phenyl or phenyl-lower alkyl group,
to obtain a compound of formula (I/b) : 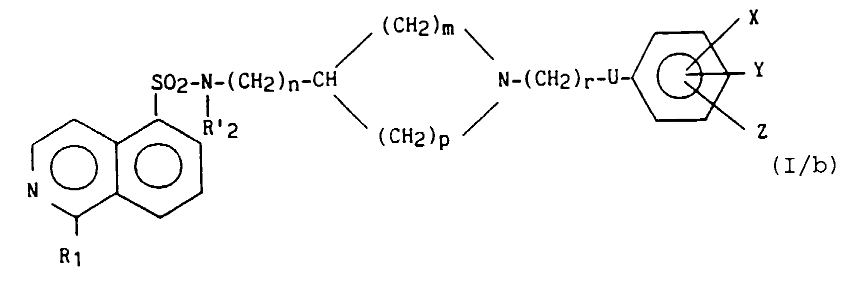 wherein R₁, X, Y, Z, U, n, m, p, r and R'₂ are as defined above, the totality of the compounds of formulae (I/a) and (I/b) forming the totality of the compounds of the general formula (I), which compounds of formulae (I/a) and (I/b) are separated, where appropriate, into their isomers and converted into salts, if desired, with a pharmaceutically acceptable acid.

2. A process for the preparation of compounds (I) corresponding more precisely to formula I/c : 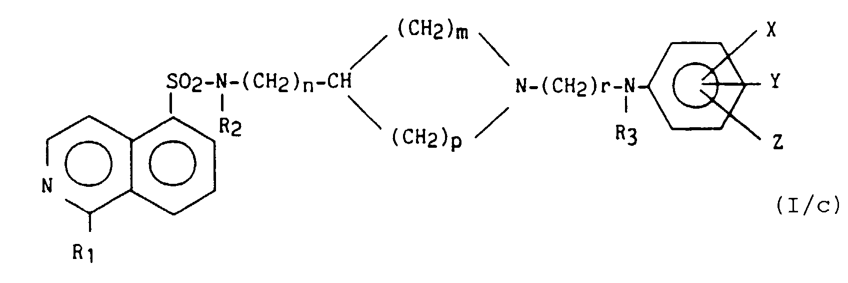 wherein R₁, R₂, R₃, X, Y, Z, n, m, p and r are as defined in claim 1,
characterised in that
- an amine of formula (II/b) : 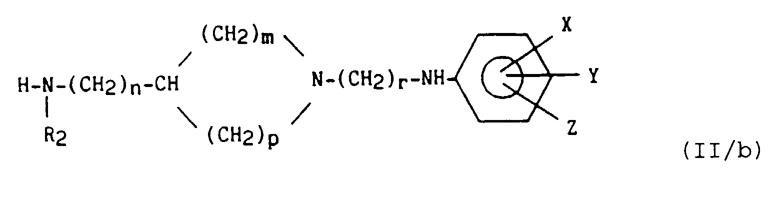 wherein R₂, X, Y, Z, n, m, p and r are as defined in claim 1, is condensed
- with a compound of formula (III) 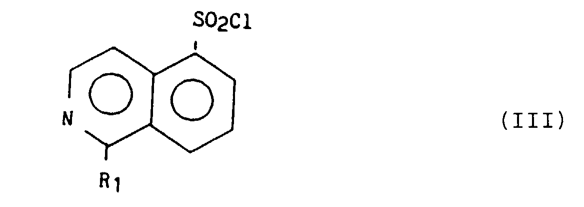 wherein R₁ is as defined in claim 1,
to obtain a compound of formula (I/d) : 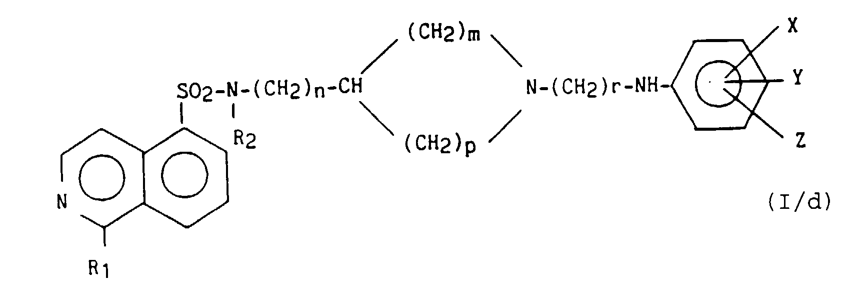 wherein R₁, R₂, X, Y, Z, n, m, p and r are as defined above,
which compound of formula (I/d) is substituted on the amine by a group of formula R'₃, wherein R'₃ has the same meanings as R₃ with the exception of the hydrogen atom, to obtain a compound of formula (I/e) : 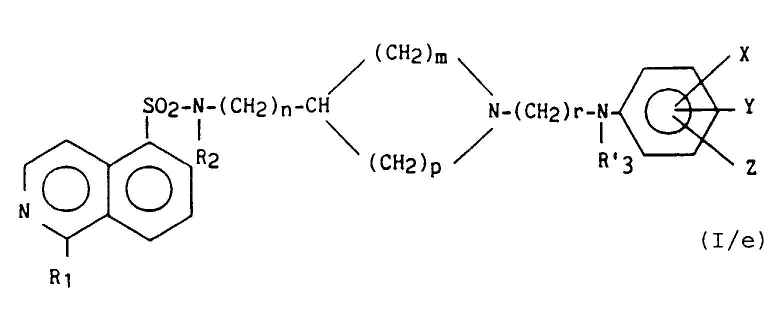 wherein R₁, R₂, X, Y, Z, n, m, p, r and R'₃ are as defined above,
- which compound of formula (I/e) is separated, where appropriate, into its isomers, and converted into a salt, if desired, with a pharmaceutically acceptable acid.
